# EUROPÄISCHE PATENTANMELDUNG

(11) **EP 4 086 917 A1**
(43) Veröffentlichungstag der Anmeldung: **09.11.2022**
(21) Anmeldenummer: 21171917.4
(22) Anmeldetag: 04.05.2021
(51) Int. Cl.: G16H 30/20, G16H 30/40

(54) **COMPUTERIMPLEMENTIERTES VERFAHREN ZUR ANALYSE VON MEDIZINISCHEN 3D-BILDDATEN, VERWENDUNG DES VERFAHRENS, COMPUTERPROGRAMM UND SYSTEM ZUR ANALYSE VON MEDIZINISCHEN 3D-BILDDATEN**

(71) Anmelder: Scholz, Alexander, 53474 Bad Neuenahr-Ahrweiler (DE)
(72) Erfinder: KELLER, René, 50858 Köln (DE)
(74) Vertreter: Müller Schupfner & Partner Patent- und Rechtsanwaltspartnerschaft mbB

(57) **Zusammenfassung**

Es wird ein computerimplementiertes Verfahren zur Analyse von medizinischen Bilddaten mit einer webbasierten Anwendung auf einem Client-Gerät (10) bereitgestellt. Das Verfahren umfasst:
a) Bereitstellen von hochaufgelösten medizinischen 3D-Bilddaten auf einem Serversystem (1),
b) Verarbeiten der hochaufgelösten 3D-Bilddaten in Abhängigkeit von Leistungsparametern des Client-Geräts (10), um datenreduzierte Bilddaten zu erlangen,
c) Übermitteln der datenreduzierten Bilddaten an das Client-Gerät (10),
d) Erzeugen einer Visualisierung der datenreduzierten Bilddaten in dem Client-Gerät (10),
e) Erlauben einer Bearbeitung der Visualisierung der datenreduzierten Bilddaten in dem Client-Gerät (10) durch einen Nutzer, um Bearbeitungsbefehle zu erzeugen,
f) Übermitteln der Bearbeitungsbefehle von dem Client-Gerät (10) an das Serversystem (1),
g) Anwenden der Bearbeitungsbefehle auf die hochaufgelösten 3D-Bilddaten, um bearbeitete Bilddaten zu erlangen,
h) Übermitteln zumindest eines Teils der bearbeiteten Bilddaten von dem Serversystem (1) an das Client-Gerät (10).

## Beschreibung

Die vorliegende Erfindung betrifft ein computerimplementiertes Verfahren zur Analyse von medizinischen drei-dimensionalen (3D) Bilddaten mit einer webbasierten Anwendung auf einem Client-Gerät. Ferner betrifft die Erfindung eine Verwendung des Verfahrens in der Zahnmedizin, ein Computerprogramm und ein System zur Analyse von medizinischen 3D-Bilddaten.

In der Medizin werden Verfahren wie Computertomographie (CT) oder Magnet Resonanz Tomographie (MRT) als bildgebende Verfahren eingesetzt. Als Ergebnis dieser bildgebenden Verfahren werden medizinischen 3D-Bilddaten ausgegeben, insbesondere Volumendaten, die dreidimensional die untersuchten Objekte repräsentieren. Die Bilddaten werden meist in standardisierten Datenstrukturen, zumeist dem sogenannten DICOM-Format, abgespeichert und können mit unterschiedlichen Softwareprodukten visualisiert und bearbeitet werden. Diese Softwareprodukte bieten eine interaktive Bedienmöglichkeit, so dass sich Nutzer räumlich in den visualisierten Bilddaten bewegen und arbeiten können.

Die 3D-Bilddaten werden zumeist auf physischen Datenträgern (CD, DVD, etc.) gespeichert, da die Größe oft mehrere hundert Megabyte beträgt. Die Verwendung physischer Datenträger macht eine weitere Verarbeitung jedoch kompliziert, da diese vom Patienten zum Arzt und vom Arzt weiter zu anschließenden Dienstleistern, die beispielsweise die Planung von Zahn-Implantaten durchführen, gereicht werden müssen. Weiterhin erschwert zunehmend die technische Entwicklung von PC-Hardware die Nutzung externer optischer Datenträger, da optische Lesegeräte heutzutage nicht mehr in allen PCs und Notebooks verbaut sind.

Ferner sind medizinische Bilddaten aufgrund von nationaler Gesetzgebung oftmals als besonders schützenswerte Daten eingestuft. Dies impliziert, dass Unberechtigte keinen Zugriff auf diese Bilddaten erhalten dürfen. Um dies zu gewährleisten sehen nationale Gesetzgebungen eine Verschlüsselung der Bilddaten vor. Dies ist derzeit jedoch nicht immer gegeben. Unter Umständen können Unberechtigte auf die Bilddaten zugreifen und kopieren, wenn Zugang zum physischen Datenspeicher (CD, DVD, etc.) besteht.

Die physisch gespeicherten Bilddaten werden oft an einen Dienstleister, etwa ein Zahntechniklabor, weitergegeben und dort gespeichert und verändert. Die Modellierungsarbeiten werden unter Verwendung stationär installierter Softwareprodukte vorwiegend manuell von Zahntechnikern durchgeführt. Dabei haben die Softwareprodukte oft hohe Anforderungen an die verwendete Hardware, weswegen diese kostenintensiv ist. Ein Zahntechniklabor plant dann beispielsweise Zahnimplantate, die künstliche Zahnwurzeln darstellen und im Knochen des Patienten verankert werden. Dabei erstellt der Zahntechniker eine Bohrschablone unter Zuhilfenahme von medizinische Bilddaten (meist ein MRT-Bild) und stellt bei der Modellierung sicher, dass beispielsweise keine Nerven durch das Implantat verletzt werden oder Winkel und Position des Implantats so angeordnet werden, dass sie mit Bohrern durch den geöffneten Mund eingeschraubt werden können. Auf das Implantat wird ein sogenanntes Abutment gesetzt, welches das Implantat mit der Zahnkrone verbindet. Diese Abutments können abgewinkelt sein, damit die Krone in die Reihe der Bestandszähne passt, obwohl das Implantat aufgrund der räumlichen Situation einen anderen Winkel aufweist. Die Ausrichtung des Implantats und die Auswahl des Abutments ist eine intellektuelle Leistung des Zahntechnikers, die viel Erfahrung und Produktkenntnis erfordert und im Fall einer Fehlplanung zu ästhetischen und gesundheitlichen Schäden beim Patienten führen kann.

Die vorliegende Erfindung macht es sich zur Aufgabe, die oben dargestellten Probleme zu lösen, insbesondere indem ein dezentrales, web-basiertes und datenschutzkonformes Verfahren zur Analyse von medizinischen Bildinformation bereitgestellt wird.

Erfindungsgemäß wird ein computerimplementiertes Verfahren zur Analyse von medizinischen 3D-Bilddaten mit einer webbasierten Anwendung auf einem Client-Gerät mit den Merkmalen des Anspruchs 1, eine Verwendung des Verfahrens in der Zahnmedizin mit den Merkmalen des Anspruchs 13, ein Computerprogramm mit den Merkmalen des Anspruchs 14 und ein System zur Analyse von medizinischen 3D-Bilddaten mit den Merkmalen des Anspruchs 15 bereitgestellt.

Gemäß einem Aspekt der vorliegenden Erfindung wird ein computerimplementiertes Verfahren zur Analyse von medizinischen 3D-Bilddaten mit einer webbasierten Anwendung auf einem Client-Gerät bereitgestellt, wobei das Verfahren umfasst:
a) Bereitstellen von hochaufgelösten medizinischen 3D-Bilddaten auf einem Serversystem,
b) Verarbeiten der hochaufgelösten 3D-Bilddaten in Abhängigkeit von Leistungsparametern des Client-Geräts, um datenreduzierte Bilddaten zu erlangen,
c) Übermitteln der datenreduzierten Bilddaten an das Client-Gerät,
d) Erzeugen einer Visualisierung der datenreduzierten Bilddaten in dem Client-Gerät,
e) Erlauben einer Bearbeitung der Visualisierung der datenreduzierten Bilddaten in dem Client-Gerät durch einen Nutzer, um Bearbeitungsbefehle zu erzeugen,
f) Übermitteln der Bearbeitungsbefehle von dem Client-Gerät an das Serversystem,
g) Anwenden der Bearbeitungsbefehle auf die hochaufgelösten 3D-Bilddaten, um bearbeitete Bilddaten zu erlangen,
h) Übermitteln zumindest eines Teils der bearbeiteten Bilddaten von dem Serversystem an das Client-Gerät.

Gegenüber bekannten Verfahren ist es bei der vorliegenden Erfindung nicht mehr notwendig, die hochaufgelösten medizinischen 3D-Bilddaten vollständig auf das Client-Gerät zu laden, mit dem die 3D-Bilddaten visualisiert und/oder bearbeitet werden, um eine Bearbeitung von 3D-Bilddaten beginnen zu können. Gemäß einem Aspekt der Erfindung wird auf dem Client-Gerät lediglich eine Web-Anwendung ausgeführt, die eine Visualisierung und/oder eine Bearbeitung von 3D-Bilddaten (beispielsweise Volumendaten, wie beispielsweise Voxel) ermöglicht. Die 3D-Bilddaten können beispielsweise mit einem MRT einem CT oder einem Ultraschallgerät erzeugt werden und eine Größe von 128 bis 2056³ Voxel aufweisen. Dabei kann ein Voxel eine Kantenlänge von 0,1 bis 0,5 mm aufweisen. Ferner können die Bilddaten auch vierdimensionale Daten sein, wobei die 4. Dimension die Zeit ist. Aufgrund der Nutzung einer Web-Anwendung ist keine Installation einer Visualisierungssoftware und/oder Bearbeitungssoftware auf dem Client-Gerät notwendig. Damit können auch leistungsschwache Client-Geräte genutzt werden. Die zur Visualisierung der 3D-Bildaten notwendigen Information können dynamisch und situationsangepasst (d.h. basierend auf Leistungsparametern; Details hierzu folgen weiter unten) von dem Serversystem zur Verfügung gestellt werden. Ferner kann unter bestimmten Umständen eine rechenaufwendige Bearbeitung durch das Serversystem durchgeführt werden und nicht durch das Client-Gerät. Dabei werden die hochaufgelösten medizinischen 3D-Bilddaten zumindest zu Beginn einer Bearbeitung vollständig lediglich auf dem Serversystem zur Verfügung gestellt. Basierend auf den Leistungsparametern können mittels einer bidirektionalen asynchronen Schnittstelle Daten zwischen dem Client-Gerät und dem Serversystem ausgetauscht werden, so dass zum einen eine Visualisierung auf dem Client-Gerät realisiert werden kann und zum anderen die hochaufgelösten 3D-Bilddaten auf dem Serversystem mittels von dem Client-Gerät übersandten Bearbeitungsbefehlen bearbeitet werden können.

Die Erfindung stellt somit eine verteilte Architektur für die Analyse von medizinische 3D-Bilddaten bereit, mit einem Teil, der in einer Serverumgebung (d.h. auf dem Serversystem) laufen kann, die in der Regel eine kontrollierte, sichere Umgebung ist. Das Verfahren kann auf dem Serversystem beispielsweise durch einen Prozess, einen Thread in einem Prozess, eine virtuelle Maschine oder eine Cloud-Instanz ausgeführt werden. Der andere Teil der verteilten Architektur kann auf dem Client-Gerät (beispielsweise einem persönlichen Gerät eines Nutzers) laufen, z. B. innerhalb eines Internetbrowsers, der auf einem PC, Tablet oder Smartphone (d.h. eine Webanwendung), oder unter Verwendung einer anderen Laufzeitumgebung oder eines Auswerters (z. B. Java, Ruby, Python), oder als Anwendung für mobile Geräte ("mobile Anwendung" oder "App"), die über Kanäle wie Apples AppStore, Googles GooglePlay oder über das Intranet verfügbar sind. Das Serversystem kann eine erste Recheneinheit und das Client-Gerät kann eine zweite Recheneinheit aufweisen. Dabei kann das erfindungsgemäße Verfahren teilweise von der ersten Recheneinheit und teilweise von der zweiten Recheneinheit ausgeführt werden. Für den Nutzer funktionieren die beiden Teile jedoch wie eine virtuelle Einzelanwendung. Vorzugsweise werden die Schritte a), b), c), g) und optional h) von dem Serversystem ausgeführt und die Schritte d), e), f) von dem Client-Gerät. Dadurch kann eine Bearbeitung von hochaufgelösten 3D-Bilddaten schnell und effizient durchgeführt werden.

Zur zweckbezogenen Verarbeitung von Bilddaten kann der jeweilige Eigentümer der Daten (beispielsweise ein Patient, von dem beispielsweise MRT-Daten als die hochaufgelösten 3D-Bilddaten erstellt worden sind) Dienstleitern die Weiterverarbeitung und/oder Modifikation der 3D-Bilddaten erlauben (beispielsweise einem Zahntechniklabor). Dazu kann das Verfahren einen Freigabeschritt umfassen, der die Art und Umfang der dem Client-Gerät zur Verfügung gestellten Bilddaten bestimmt. Ferner kann der Schritt b) ein Anpassen der Bilddaten an die erteilte Freigabe des Eigentümers umfassen. Gemäß einem Aspekt der Erfindung wird, da die jeweiligen Bilddaten im Original grundsätzlich für mehrere Anwendungsfelder genutzt werden können, jede Modifikation der Bilddaten als gesondertes Ereignis zu einem Ursprungsdatensatz der 3D-Bilddaten verwaltet und/oder gespeichert, ohne dass der Ursprungsdatensatz geändert wird. Ist beispielsweise für ein Zahntechniklabor nur der Kieferbereich eines Kopf-MRTs relevant, können in Schritt a) die 3D-Bilddaten auf Basis von Verwendungsinformationen beschnitten werden, um eine bessere Übersichtlichkeit und Performance für den Nutzer zu erhalten. Der Eigentümer hat jedoch immer noch Zugriff auf die ursprünglichen 3D-Bilddaten.

Die hochaufgelösten 3D-Bilddaten können zudem Geometriedaten von künstlichen Strukturen wie Implantate und/oder Abdrücke umfassen. Dabei kann es sich um Modelle handeln, die an den Patienten angepasst sind oder an diesen anzupassen sind. Beispielsweise kann ein Zahntechniklabor ein Implantat erstellen und es zusammen mit MRT oder CT Bilddaten des Kiefers, für den es erstellt wurde, als die hochaufgelösten 3D-Bilddaten abspeichern. Ferner kann ein Abdruck eines Gebisses von einem Arzt erzeugt werden und zusammen mit den MRT oder CT Bilddaten als die hochaufgelösten 3D-Bilddaten abgespeichert sein. Insbesondere in der Zahnmedizin ist es vorteilhaft, insbesondere Zahnimplantate (als Geometriedaten) zusammen mit den Bilddaten eines Patienten darzustellen. Somit kann der Planungsablauf vereinfacht und beschleunigt werden.

Die Leistungsparameter können bei einem Start der Webanwendung in dem Client-Gerät durch einen Performance-Test bestimmt werden, indem beispielsweise die Hardware, Software und/oder die Internetanbindung des Client-Geräts analysiert wird. Im Fokus stehen insbesondere die Geschwindigkeit der Grafikkarte im Hinblick auf 3D-Visualisierung, insbesondere Polygone, der installierte Webbrowser und Skriptsprache die Auflösung eines Bildschirms sowie die Upload- und Downloadgeschwindigkeit. Mit diesen Leistungsparametern kann dann der Datenaustausch zwischen Serversystem und Client-Gerät konfiguriert werden (Verbindungskonfiguration). Somit kann ein situationsangepasster Datenaustausch zwischen Serversystem und Client-Gerät bewirkt werden, um die Bearbeitungsgeschwindigkeit zu erhöhen. Demgemäß kann das Verfahren vor Schritt b) die folgenden Schritte umfassen: Bestimmen von Leistungsparameter des Client-Geräts, wobei die Leistungsparameter vorzugsweise Informationen über Hardware, Software und/oder einer Verbindung zwischen Client-Gerät und Serversystem umfassen, und Übermitteln der Leistungsparameter an das Serversystem.

Gemäß einem Aspekt der vorliegenden Erfindung hat das Serversystem die Aufgabe die hochaufgelösten 3D-Bilddaten zu verwalten und/oder zu bearbeiten und dem Client-Gerät basierend auf den Leistungsparametern in bearbeiteter Form zumindest teilweise zur Verfügung zu stellen. Ferner hat das Client-Gerät die Aufgabe die von dem Serversystem empfangenen Daten (d.h. die datenreduzierten Bilddaten) zu visualisieren und einem Nutzer darzustellen. Ferner kann das Client-Gerät Bearbeitungsbefehle von dem Nutzer aufnehmen und auf die datenreduzierten Bilddaten anwenden und/oder an das Serversystem übermitteln. Somit ist eine bidirektionale Verbindung zwischen Client-Gerät und Serversystem bereitgestellt, so dass von beiden Seiten (d.h. serverseitig und clientseitig) Informationen gesendet und empfangen werden können, insbesondere parallel, um die Bearbeitungsgeschwindigkeit weiter zu erhöhen. Mit anderen Worten können Bilddaten in dem Client-Gerät und in dem Serversystem gleichzeitig bearbeitet werden. Auch eine Bearbeitung der 3D-Bilddaten oder der datenreduzierten Bilddaten, die von dem Serversystem und/oder dem Client-Gerät durchgeführt werden, können gleichzeitig ausgeführt werden. Somit kann eine erhöhte Bearbeitungsgeschwindigkeit erzielt werden, da nicht erst auf eine Antwort der anderen Seite gewartet werden muss.

Die hochaufgelösten medizinischen Bilddaten werden beispielsweise als Ergebnis von Computertomographie (CT) oder Magnetresonanztomographie (MRT) generiert. Die Größe dieser Daten beträgt oft mehrere Hunderte Megabyte, sodass ein vollständiges herunterladen der Daten von dem Serversystem langwierig ist. Ferner ist die Größe der Daten oft ein Problem, da diese nicht vollständig in den Speicher der Webanwendung (beispielsweise eines Browsers) geladen werden können. Bei der oben dargelegten Architektur können die 3D-Bilddaten zentral auf dem Serversystem bereitgestellt (Schritt a)) werden und beispielsweise über das Internet an das Client-Gerät übermittelt werden. So können sich das Serversystem und das Clientgerät an unterschiedlichen Orten befinden. Das einfache Handhaben von großen Datenmengen ist besonders in der Zahnmedizin von Vorteil, da es hier regelmäßig mit umfangreichen Bilddaten gearbeitet wird (beispielsweise bei der Planung von Implantaten, bei der Planung von Operationen usw.).

Daraufhin können im Verfahrensschritt b) die hochaufgelösten 3D-Bilddaten in Abhängigkeit von den Leistungsparametern so verarbeitet werden, dass datenreduzierte Bilddaten erlangt werden. Bei den datenreduzierten Bilddaten kann es sich beispielsweise um Bilddaten mit einer geringeren Auflösung als im Vergleich zu den hochaufgelösten 3D-Bilddaten handeln. Die Verarbeitung der 3D-Bilddaten kann also eine Reduzierung eines Datenvolumens der hochaufgelösten 3D-Billdaten sein. Diese Reduzierung kann variabel in Abhängigkeit der Leistungsparameter durchgeführt werden. So kann beispielsweise ein Bereich, der in den hochaufgelösten 3D-Bilddaten durch 128 Voxel definiert ist, in den datenreduzierten Bilddaten durch 16 Voxel definiert sein. Somit kann eine Auflösung der Bilddaten reduziert werden. Neben den Bilddaten können auch weitere Informationen gespeichert werden, die den Bilddaten insgesamt oder nur einem bestimmten Bereich der Bilddaten zugeordnet sein können. Dies können beispielsweise eine Farbinformation, Attribute, Produktinformationen, Patienteninformationen, Informationen über die verwendete Modalität usw. sein. Diese weiteren Informationen können zusätzlich oder alternativ zumindest teilweise entfernt werden, um die datenreduzierten Bilddaten zu erzeugen. Ferner kann die Verarbeitung ein Wegschneiden von bestimmten Bereichen der hochaufgelösten 3D-Bilddaten sein. Somit kann die Verarbeitung der hochaufgelösten 3D-Bilddaten ein Freistellen bestimmter Bereiche (z.B. eines Bereichs von Interesse) sein. Mit anderen Worten können die datenreduzierten Bilddaten einen geringeren Speicherplatzbedarf aufweisen, als die hochaufgelösten 3D-Bilddaten. Die datenreduzierten Bilddaten können 3D-Bilddaten (wie beispielsweise Volumendaten, Oberflächendaten usw.) oder 2D-Bilddaten (wie beispielsweise Schnitte, Multiplanar Reformation MPR-Ansichten, Ansichten usw.) sein. Sie können auch Oberflächenmodelle sein oder enthalten, z.B. der aus den 3D-Bilddaten segmentierten Zahnoberfläche, die z.B. als Drahtgittermodell definiert sein kann. Die datenreduzierten Bilddaten können nach deren Erstellung auf dem Serversystem gespeichert werden. Somit können die datenreduzierten Daten für eine Anfrage verfügbar sein und müssen dann nicht erneut erzeugt werden. Dies reduziert die vom Serversystem benötigte Rechenleistung und sorgt dafür, dass die datenreduzierten Bilddaten schnell verfügbar sind. Die datenreduzierten Bilddaten können einen geringeren Speicherbedarf aufweisen als die hochaufgelösten 3D-Bilddaten. In der Zahnmedizin können die Zahnoberfläche und/oder die Knochenoberfläche vorteilhaft als Oberflächenmodell dargestellt werden. Dabei kann Gewebe als Volumengrafik dargestellt werden. Dadurch kann eine Planung beispielsweise eines Implantats hochgenau durchgeführt werden, ohne dass die gesamten Bilddaten als Volumengrafik dargestellt sein müssen. Dies erleichtert und beschleunigt die Planungsphase.

In Schritt c) können die datenreduzierten Bilddaten an das Client-Gerät übermittelt werden. Aufgrund des reduzierten Datenvolumens der Bilddaten, kann die Übermittlung schnell gehen, so dass der Nutzer kurz nach einem Start der Webanwendung mit der Arbeit beginnen kann. Somit kann ein zügiges Arbeiten erlaubt werden, ohne dass erst lange auf eine Übertragung der Bilddaten gewartet werden muss.

Gemäß einem Aspekt der Erfindung kann in Abhängigkeit der Verbindungskonfiguration das Serversystem ein vereinfachtes Modell (d.h. die datenreduzierten Bilddaten) der hochaufgelösten 3D-Bilddaten und/oder Geometriedaten, wie STL (Stereo Lithographie Format) oder PLY (Polygon File Format) an das Client-Gerät übermitteln. Das STL-Format beschreibt die Oberfläche von 3D-Körpern mithilfe von Dreiecksfacetten. Jede Dreiecksfacette wird durch die drei Eckpunkte und die zugehörige Flächennormale des Dreieckes charakterisiert. Sind die drei Eckpunkte beim Blick auf eine Dreiecksfläche gegen den Uhrzeigersinn angeordnet, wird die Fläche als Außenseite eines Volumenkörpers (Solid) angenommen. Das PLY-Format zeichnet sich durch eine einfache Beschreibung einzelner Objekte als Listen von Polygonen aus. Für Vorder- und Rückseite eines Polygons können unterschiedliche Attribute definiert werden, wie zum Beispiel: Farbe, Transparenz, Oberflächen-Normalen, Textur-Koordinaten oder Konfidenz-Werte gemessener Daten. Die redundante Flächennormale weist immer aus dem Körper heraus. Das Client-Gerät kann zusätzlich Metadaten der Bilddaten empfangen, die Informationen über die datenreduzierten Bilddaten enthalten und welche gröberen und feineren Bilddaten mit welchen Datenvolumen serverseitig angeboten werden (z.B. aus vorherigen Bearbeitungen). In Abhängigkeit der Leistungsparameter kann das Client-Gerät automatisch oder durch eine Nutzereingabe entscheiden, welche Bilddaten asynchron nachgeladen werden. Insbesondere könnten gröbere, speicherärmere Varianten abgerufen werden, um eine Visualisierung bei geringer Leistung des Client-Geräts (insbesondere der Grafikkarte) während interaktiven Drehoperationen darzustellen. Feinere Varianten können asynchron nachgeladen werden, wenn die Internetanbindung langsam ist, die Hardware des Nutzers aber leistungsfähig genug für eine Visualisierung detailreicherer Bilddaten ist. Asynchron kann dabei bedeuten, dass das Client-Gerät nicht auf vollständige Daten wartet, sondern im Hintergrund (d.h. während die Bearbeitung an dem Client-Gerät fortgesetzt wird) Daten sukzessive nachlädt.

Sind die Bilddaten an das Client-Gerät übermittelt, so kann das Client-Gerät in Schritt d) aus den datenreduzierten Bilddaten eine Visualisierung erstellen. Die Visualisierung kann ein Volumenrendering oder ein Oberflächenrendering sein. Welches Rendering verwendet wird, kann von den zuvor bestimmten Leistungsparametern abhängen, insbesondere von der Leistungsfähigkeit des Client-Geräts und den zur Verfügung gestellten datenreduzierten Bilddaten. Werden aufgrund einer schlechten Verbindung mit dem Serversystem nur wenige Bilddaten übermittelt, so kann ein Oberflächenrendering genutzt werden, da so zügig eine Visualisierung bereitgestellt werden kann. Bei einem Oberflächenmodell kann es sich um eine 3D-Grafik handeln. Hier werden die in der Grafik sichtbaren Flächen in Form von farbigen Polygonen dargestellt, welche miteinander verbunden sind. Der Grundlage für ein solches Flächenmodell ist ein Drahtmodell (auch als Kantenmodell bezeichnet). Dort hat jedes einzelne Polygon eine ihm genau zugeordnete Farbe. Ein solches Vieleck (Polygon) ist durch Kanten verbunden und wird auch durch seine Eckpunkte beschrieben. Demgegenüber kann Volumenrendering eine Volumengrafik erzeugen, so dass transparente Objekte - z. B. lichtdurchlässiges Hautgewebe - und Objekte ohne scharfe Abgrenzungen - wie z. B. Wolken - mit hoher Genauigkeit und Wirklichkeitstreue bildlich dargestellt werden können. Sie ist für bildgebende Verfahren von großer Bedeutung, da diese von Natur aus Voxeldaten erzeugen. Liegen mehr Bilddaten vor und ist das Client-Gerät leistungsfähig genug, kann ein Volumenrendering genutzt werden, so dass dem Nutzer relativ zum Oberflächenmodell viele Informationen zur Verfügung stehen. Das Client-Gerät kann also eine Volumengrafik und/oder ein Oberflächenmodell aus den datenreduzierten Bilddaten erstellen. Somit kann eine vereinfachte Darstellung der hochaufgelösten 3D-Bilddaten auf dem Client-Gerät erstellt werden, da die datenreduzierten Bilddaten basierend auf den individuellen Leistungsparametern des Client-Geräts angepasst sind. Dadurch ist ein flüssiges Arbeiten bzw. ein schnelles Bereitstellen der Visualisierung der Bilddaten in dem Client-Gerät möglich. Parallel zu der Visualisierung der datenreduzierten Bilddaten können weitere Bilddaten von dem Serversystem heruntergeladen werden, die mehr Informationen umfassen als die zuerst heruntergeladenen datenreduzierten Bilddaten. Dies kann basierend auf den Leistungsparametern erfolgen. Parallel dazu können detailliertere (d.h. hochaufgelöstere) datenreduzierte Bilddaten von dem Serversystem nachgeladen werden, die im Vergleich zu den hochaufgelösten 3D-Bilddaten immer noch eine geringere Auflösung aufweisen aber eine höhere Auflösung aufweisen als die zuerst geladenen datenreduzierten Bilddaten. Durch das parallele Nachladen kann ein flüssiges Arbeiten für den Nutzer sichergestellt werden, ohne dass der Nutzer in seiner Bearbeitung der Visualisierung eingeschränkt ist. Das Nachladen von weiteren Daten von dem Serversystem kann auch von dem Nutzer aktiv gesteuert werden. So kann dem Nutzer angezeigt werden, welche weiteren vereinfachten Versionen des hochaufgelösten 3D-Bilddaten auf dem Serversystem zur Verfügung stehen, sodass der Nutzer auswählen kann, welche Daten parallel nachgeladen werden sollen.

Sobald detailliertere Bilddaten in dem Client-Gerät zur Verfügung stehen, kann die bereits dargestellte Visualisierung angepasst und zumindest teilweise durch eine Visualisierung der neu zur Verfügung stehenden Bilddaten ersetzt werden. So kann die Visualisierung einen hochaufgelösten Bereich und einen relativ dazu niedrigaufgelösten Bereich umfassen. Beispielsweise kann in der Visualisierung in dem Client-Gerät der hochaufgelöste Bereich als Volumenmodell dargestellt sein und der niedrigaufgelöste Bereich als Oberflächenmodell. Somit kann ein Bereich von Interesse detaillierter dargestellt sein als der Rest der Visualisierung, wobei dennoch das Gesamtmodell dargestellt ist, um dem Nutzer eine ganzheitliche Visualisierung anzuzeigen.

Die Geschwindigkeit bei der Erstellung der Visualisierung von dreidimensionalen Daten hängt insbesondere von dem Speicher und der Grafikkarte des Client-Geräts ab. Bei Webanwendungen, die im Browser laufen, ist zudem ein Performance-Faktor für die Berechnungsgeschwindigkeit die verwendete Programmiersprache. Derzeit kommt vorwiegend JavaScript zum Einsatz, wenn eine clientseitige Dynamik in einer Webanwendung realisiert werden soll. JavaScript ist dabei eine Skriptsprache und hinsichtlich der Ausführungsgeschwindigkeit langsamer als vorkompilierte oder kompilierte Programme. Neuere Entwicklungen von Skiptsprachen, wie TypeScript oder auch die Möglichkeit von vorkompilierten Programmen in einer Webanwendung, insbesondere WebAssembly, erlangen zunehmend Verbreitung in neuen Webanwendung (z.B. Browsern). Das erfindungsgemäße Verfahren kann hierbei einen Programmcode für performance-kritische Berechnungen in unterschiedlichen Realisierungsvarianten bereitstellen. Verfügt das Client-Gerät beispielsweise über einen modernen Browser der die Ausführung von vorkompilierten Programmen unterstützt, werden die datenreduzierten Bilddaten in einem Speicherbereich der Webanwendung gespeichert, auf das jede Skiptsprachen (JavaScript und WebAssembly) gleichermaßen zugreifen kann, sodass die performancekritischen Berechnungen beispielsweise mittels WebAssembly durchgeführt werden können, was deutliche Performancevorteile bringt. Es können jedoch auch andere Programmiersprachen durch das erfindungsgemäße Verfahren genutzt werden.

Die datenreduzierten Bilddaten, die in dem Client-Gerät visualisiert werden können, können auch Geometriedaten wie STL-Daten (Stereolithographie) oder PLY-Daten (polygon file format) umfassen. Die Geometriedaten können von Nutzern zuvor erstell worden sein und auf dem Serversystem gespeichert sein. Die Geometriedaten können dabei Implantate, Gebissabdrücke darstellen, die in Verbindung mit den medizinischen Bilddaten analysiert werden sollen. Somit kann eine interdisziplinäre Bearbeitung der medizinischen Bilddaten besonders einfach möglich sein, da Arbeitsresultate wie Geometriedaten in Abhängigkeit der jeweiligen Leistungsparameter schnell und effizient ausgetauscht werden können.

Gemäß einem Aspekt der Erfindung wird die Visualisierung dem Nutzer im Schritt e) so zur Verfügung gestellt, dass der Nutzer die Visualisierung der datenreduzierten Daten bearbeiten kann, um Bearbeitungsbefehle zu erzeugen. Durch das Bearbeiten des Nutzers können Bearbeitungsbefehle erzeugt werden, die insbesondere die Art der Bearbeitung und den Ort der Bearbeitung umfassen. Bearbeitungsbefehle können Programmanweisungen sein, die durch eine Benutzerinteraktion ausgelöst werden und in der Regel zu einer Modifikation der Bilddaten (entweder der datenreduzierten Bilddaten oder der hochaufgelösten Bilddaten) durch Modifikationsalgorithmen führen (dies sind beispielsweise Glätten, Zuschneiden, etc.). Ferner können Bearbeitungsbefehle auch durch Hinzufügen von Informationen zu den Bilddaten erzeugt werden. Beispielsweise kann ein Nutzer die Visualisierung durch Metadaten, geometrischen Modellen oder anderen Informationen anreichern (z.B. eine Platzierung und Ausrichtung einer Krone für ein Zahnimplantat) So kann der Nutzer beispielsweise die Visualisierung zurechtschneiden, sodass die Bearbeitungsbefehle Informationen darüber enthalten, wo der Schnitt verläuft und welcher Teil der Visualisierung weggeschnitten werden soll. Die Bearbeitungsbefehle können dann direkt auf die datenreduzierten Bilddaten in dem Client-Gerät angewendet werden und/oder an das Serversystem übermittelt werden. Im ersten Fall, kann das Client-Gerät die datenreduzierten Bilddaten selbst verarbeiten, ohne dass das Serversystem bei der Bearbeitung involviert ist. Zusätzlich können die Bearbeitungsbefehle an das Serversystem übermittelt werden (Schritt f)). Dort können die Bearbeitungsbefehle auf die hochaufgelösten 3D-Bildinformationen angewendet werden (Schritt g)). Zusätzlich können die Bearbeitungsbefehle auf dem Serversystem gespeichert werden, um die Bearbeitungen eines Nutzers später nachvollziehen zu können. Vorzugsweise werden alle Bearbeitungsinteraktionen (d.h. Bearbeitungsbefehle) protokolliert, so dass das Client-Gerät (z.B. die Webanwendung) die den Bearbeitungsbefehle zugeordneten Modifikationsalgorithmen im Client-Gerät auf die datenreduzierten Bilddaten anwenden kann. Gleichzeitig werden die Bearbeitungsbefehle parallel an das Serversystem übertragen, wo sie auf die hochaufgelösten Bilddaten angewendet werden können. Somit kann das Hin- und Rückübertragen von bearbeiteten Bilddatenminimiert und/oder verringert werden. Dies ist insbesondere nützlich in dem Fall, bei dem das Client-Gerät eine hohe Leistung aufweist aber die Verbindung zu dem Serversystem schlecht ist.

Grundsätzlich gibt es vielfältige Operationen zur Modifikation von Bilddaten wie Volumendaten oder Geometriedaten, wie Zuschneiden oder Glätten-Operationen. Einige dieser Verfahren sind trivial, weitere Verfahren sind in gängiger Literatur beschrieben und frei verfügbar. Gewisse Verfahren sind jedoch individuell entwickelt und somit besonders schützenswert. Da die Webanwendung im Client-Gerät des Nutzers läuft, würde auch der Quelltext der geschützten Verfahren in Form von Skriptsprachen oder vorkompiliertem Programmcode in den Speicher des Client-Geräts des Nutzers geladen werden, wo er dekompiliert, analysiert und kopiert werden könnte. Daher ist es gemäß einem Aspekt der Erfindung vorgesehen, dass Operationen zur Modifikation von Volumen- und Geometriedaten ausschließlich serverseitig von dem Serversystem berechnet werden können. Dazu prüft die Softwarearchitektur bei der Anwendung einer Operation (d.h. bei Erstellung eines Bearbeitungsbefehls, Schritt e)), ob eine serverseitige Ausführung vorgesehen ist und leitet den Bearbeitungsbefehl, etwa die Bewegung mit gedrückter Maustaste über einen bestimmten Bereich der Visualisierung, an das Serversystem weiter (bspw. über eine bidirektional asynchrone Schnittstelle). Das Serversystem verarbeitet den Bearbeitungsbefehl und wendet ihn auf die hochaufgelösten 3D-Bilddaten an und sendet das Ergebnis der durch die Modifikation geänderten Bilddaten zurück an das Client-Gerät (vorzugsweise per asynchroner Kommunikation). Dabei wird die Anbindung der Internetgeschwindigkeit berücksichtigt, die ggf. nicht alle Teilergebnisse einer Modifikationsfolge (etwa 5 Sekunden langes Mausbewegen in der Operation Glätten erzeugt alle 50 ms eine neue Version) übertragen kann. In diesem Fall werden nur so viele Teilergebnisse übermittelt, wie hinsichtlich der Datenrate übertragen werden können, wobei immer das zum Zeitpunkt einer Übertragung neueste Teilergebnis übermittelt wird und Zwischenergebnisse verworfen werden können.

Das obige kann auch dann angewendet werden, wenn Modifikationen auf eine Visualisierung von datenreduzierten Bilddaten angewendet werden. Dabei können die datenreduzierten Bilddaten aufgrund der Leistungsparameter nicht dieselbe Auflösung (d.h. dieselbe Informationsdichte) wie die hochaufgelösten 3D-Bildinformationen auf dem Serversystem haben. In so einem Fall wird jedoch parallel zur serverseitigen Bearbeitung eine Algorithmik zur Durchführung der Modifikationen im Client-Gerät (d.h. in der Webanwendung) aufgerufen, damit der Nutzer ein flüssiges, unmittelbares Feedback erhält. Die aufgerufene Algorithmik kann dazu ausgestaltet sein, die Bearbeitungsbefehle eines Nutzers in dem Client-Gerät auf die datenreduzierten Bilddaten anzuwenden. Die Bearbeitungsbefehle werden parallel zur Anwendung in dem Client-Gerät an das Serversystem gesendet und dort auf den hochaufgelösten 3D-Bildinformationen angewendet. In regelmäßigen Abständen, spätestens nach Beendigung der Operationsfolge zur Erzeugung von Bearbeitungsbefehlen, werden die in den hochaufgelösten 3D-Bildinformationen geänderten Bereiche in die Auflösung der in der Webanwendung befindlichen Visualisierung umgerechnet und als die bearbeiteten Bilddaten an das Client-Gerät gesendet. Die geänderten Bereiche der bearbeiteten Bilddaten werden dann in dem Client-Gerät visualisiert und können die bisherige Visualisierung teilweise oder vollständig ersetzen.

Wenn keine serverseitige Bearbeitung der Bilddaten vorgesehen ist und die Leistung des Client-Gerät es zulässt, können Bearbeitungsbefehle auch direkt auf die Bilddaten in dem Client-Gerät angewendet werden. Dies ist beispielsweise in dem Fall vorteilhaft, wenn die Internetverbindung, mit der das Client-Gerät mit dem Serversystem verbunden ist, schlecht ist und die Leistung des Client-Geräts ausreichend gut für die Bearbeitung der Bilddaten geeignet ist. So ist eine zügige Bearbeitung möglich, ohne dass der Nutzer auf Rückmeldung von dem Serversystem warten muss. Parallel zu der Anwendung der Bearbeitungsbefehle, können diese auch an das Serversystem übertragen werden und dort auf die hochaufgelösten 3D-Bilddaten angewendet werden. Die damit erzeugten bearbeiteten Bilddaten können dann abhängig von den Leistungsparametern an das Client-Gerät übermittelt und dort visualisiert werden.

Durch die oben beschriebene Modifikation der Bilddaten, wie beispielsweise einem Zuschneiden von hochaufgelösten 3D-Bilddaten und einer Erstellung und Modellierung von Geometriedaten etwa für Zahnprothesen, wird eine Vielzahl von Zwischenzustände von einem Beginn der Modifikation bis zum Abschluss der Modifikation erstellt. Gemäß einem Aspekt der Erfindung wird je nach veränderten Bilddaten die Unterschiede einer Transaktion von beispielsweise Bearbeitungsbefehlen und/oder bearbeiteten Bilddaten berechnet und in einer speicherplatzoptimalen Variante abgelegt. Erfolgt beispielsweise der Zuschnitt der hochaufgelösten 3D-Bilddaten (z.B. eines Volumendatensatzes), wird der entfallene dreidimensionale Bereich als transparente Maske beschrieben, die so dann bei der Visualisierung der Bilddaten angezeigt werden kann. Das bedeutet im Falle eines rechteckigen Zuschnitts, bei dem ein Kopf-MRT von oben bis zum Oberkiefer zugeschnitten wird, dass lediglich drei Vektoren für das maskierende Rechteck anstelle eines neuen kompletten Volumendatensatzes gespeichert werden müssen. Bei Geometriedaten, die als Polygone gespeichert werden, kann durch eindeutige Identifikation und Zuordnung zwischen Versionen von Polygonen ebenfalls nur geänderte Teilbereiche gespeichert werden. Dieses Vorgehen ermöglicht, dass der Nutzer auch bei vielen Daten bis zu Beginn seiner Modifikation einzelne Arbeitsschritte widerrufen oder wiederherstellen kann. Gegenüber einer klassischen Speicherung von Zwischenversionen, die immer die gesamten bearbeiten Bildmaterial umfassten, kann bei dem vorliegenden Aspekt der Erfindung lediglich eine dreidimensionale Bitmaske gespeichert werden, die den nicht gewünschten Teil der Bilddaten ausblendet. Das gleiche gilt für weitere Bearbeitungsbefehle. Am Ende einer Bearbeitung könnte dann eine finale Version der bearbeitete Bilddaten als Resultat einer Anwendung vieler einzelner Bearbeitungsbefehle vorliegen und dann beispielsweise als neue Version der hochaufgelösten oder datenreduzierten Bilddaten gespeichert werden.

Somit kann ein Verfahren bereitgestellt werden, bei dem durch Bearbeiten einer Visualisierung der datenreduzierten Bilddaten dieselben Bearbeitungsbefehle auf die hochaufgelösten 3D-Bilddaten angewendet werden, ohne dass diese bei der Erstellung der Bearbeitungsbefehle direkt visualisiert und/oder bearbeitet werden müssen. Somit kann ein Bearbeitungsverfahren bereitgestellt werden, das auch auf leistungsschwachen Client-Geräten eine Bearbeitung von hochaufgelösten 3D-Bilddaten hocheffizient zulässt. Somit kann insbesondere in der Zahnmedizin, wo regelmäßig viele Beteiligte mit Bilddaten arbeiten, die Planungsphasen vereinfacht werden. Ferner ist es mit dem Verfahren leicht zusätzliche Personen einzubinden, um die Planungsqualität zu erhöhen.

In Schritt h) können die bearbeiteten hochaufgelösten 3D-Bilddaten direkt oder als datenreduzierte bearbeitete hochaufgelöste 3D-Bilddaten, die vor der Übermittlung an das Client-Gerät analog zu Schritt b) bearbeitet werden, um das Datenvolumen der bearbeiteten hochaufgelösten 3D-Bilddaten zu reduzieren, übermittelt werden. Dabei kann die Reduzierung des Datenvolumens der Reduzierung in Schritt b) entsprechen. Ferner können die bearbeiteten hochaufgelösten 3D-Bilddaten in Abhängigkeit der Leistungsparameter verarbeitet werden, um eine zügige Übermittlung an das Client-Gerät sicherstellen zu können. Die Reduzierung kann auf Basis der zuvor bestimmten Leistungsparameter oder von aktuell bestimmten Leistungsparametern ausgeführt werden.

Gemäß einem Aspekt der Erfindung werden alle Veränderungen an den Bilddaten im Serversystem revisionssicher gespeichert. Ferner kann die Speicherung verschlüsselt erfolgen, sodass die Urheberrechte an den Bilddaten entsprechende Lizenzvereinbarungen des Eigentümers gewahrt werden können. So können beispielsweise unterschiedlichen Nutzern unterschiedliche Betrachtungs- und/oder Bearbeitungsrechte an den hochaufgelösten medizinischen 3D-Bilddaten auf dem Serversystem erteilt werden. Beispielsweise kann dem Patienten dessen Bilddaten auf dem Serversystem gespeichert sind, lediglich Betrachtungsrechte eingeräumt werden, wohingegen ein betreuender Arzt die Bilddaten auch bearbeiten kann. So können beispielsweise auch Betrachtungsrechte von Geometriedaten in mehreren Stufen konfiguriert werden. Beispielsweise kann festgelegt werden, dass die Bilddaten vollständig in einem Client-Gerät angezeigt werden können (d.h. visualisiert) aber nicht herunterladbar sind. Ferner können bestimmte Geometriedaten lediglich in einer geringen Auflösung dargestellt werden oder durch das Serversystem in Volumendaten umgewandelt werden, damit eine Erstellung einer Kopie der geometrischen Daten deutlich erschwert ist. Folglich können die Zugriffsrechte und Verwertungsrechte dem jeweilig spezifischen Fall individuell angepasst werden.

Gemäß einem Aspekt der Erfindung sind alle Daten einer eindeutigen Transaktionsnummer zugeordnet. Diese Transaktionsnummer ist wiederum eindeutig einem Patienten zugeordnet. Es ist vorgesehen, dass fortgeschrittene oder qualifiziert elektronische Signaturen zur Identifikation bzw. Authentifizierung genutzt werden können. Es ist weiterhin vorgesehen, dass sämtliche Daten sicher verschlüsselt werden, in bevorzugter Ausführungsform nach einem PKI-Verfahren. Dabei wird für jeden Patienten ein eigener Schlüssel erzeugt, der nur mit einer patientenindividuellen Kennung zur Entschlüsselung der Daten verwendet werden kann. Alternativ sind insbesondere für die Erstregistrierung (beispielsweise bei einem erstmaligen Bearbeiten der Bilddaten in einem Client-Gerät) einfachere Authentifizierungsmöglichkeiten vorgesehen, die etwa eine Nennung persönlicher Daten des Patienten umfassen.

Somit kann gewährleistet sein, dass der Patient die Datenhoheit über seine medizinischen Bilddaten innehat. Vorzugsweise ist eine Genehmigung des Patienten notwendig bspw. über ein Webportal, welcher Dritte in welchem Zeitraum Zugriff auf welche Bilddaten haben darf. Weiterhin kann der Patient darüber entscheiden wie die Daten verwendet werden. Dazu kann eine Schnittstelle vorgesehen sein, über die der Patient die entsprechenden Angaben machen kann, um die Bilddaten freizugeben. So ist es beispielsweise möglich, einem Arzt ausschließlich Betrachtungsrechte zu erteilen, was in aller Regel ausreichend ist. D.h. der Arzt kann im Rahmen seines Zugriffs die Bilddaten auf seinem Client-Gerät visualisieren, aber nicht lokal speichern und/oder archivieren. Einem Zahntechniklabor hingegen könnte das Recht zum Download der Bilddaten und Upload von Geometriedaten erlaubt werden, die beispielsweise eine Simulation eines neuen Gebisses darstellen. Die Anforderung von Datenzugriffen (bspw. von einem Arzt oder einem Labor) erfolgt über die eindeutige Transaktionsnummer, die bevorzugt in Form eines QR-Codes kommuniziert werden kann. Registrierte Nutzer können, um eine Berechtigung zum Nutzen von Bilddaten von dem Patienten anzufordern, per Scan des QR-Codes bzw. Eingabe der Transaktionsnummer ein Datenzugriffsersuchen für die in dem Serversystem gespeicherten Bilddaten initiieren. Der Patient wird über dieses Zugriffsersuchen informiert, bevorzugt über eine bereitgestellte App, und sieht, wer auf welche Daten mit welcher Verwendungsintention Zugriff haben möchte. Der Patient kann über die Schnittstelle dem Ersuchen zustimmen oder es ablehnen. In beiden Fällen wird der Ersuchende darüber informiert.

Vorzugweise wird in Schritt h) zumindest ein Teil der bearbeiteten hochaufgelösten 3D-Bilddaten von dem Serversystem per asynchroner Kommunikation an das Client-Gerät übermittelt. Gemäß einem Aspekt der vorliegenden Erfindung ist eine asynchrone Kommunikation ein zeitversetztes Nachladen von Inhalten, um die Zeit zu verkürzen bis der Nutzer mit der Bearbeitung beginnen kann bzw. bei der Bearbeitung nicht behindert wird, wenn die Bilddaten noch nicht vollständig geladen wurden. Dabei können zunächst datenreduzierte Bilddaten geladen und visualisiert werden, so dass der Nutzer mit der Bearbeitung beginnen kann. Während der Bearbeitung werden im Hintergrund Bilddaten mit höherem Datenvolumen (z.B. mit einer höheren Auflösung im Vergleich zu den zuvor geladenen Bilddaten) nachgeladen. Die ursprüngliche Visualisierung der datenreduzierten Bilddaten können solange für den Nutzer sichtbar und bearbeitbar bleiben, bis die entsprechend höher aufgelösten Teile nachgeladen sind und zumindest Teile der ursprünglichen Visualisierung ersetzt haben. Ferner kann bei asynchroner Kommunikation der derzeit ausgeführte Prozess unterbrechungsfrei weiter ausgeführt werden, da beispielsweise nicht auf eine Antwort des Empfängers gewartet werden muss, wie es beispielsweise bei synchroner Kommunikation der Fall ist. Vorzugsweise ist es für die Bearbeitungsbefehle unerheblich, ob diese auf datenreduzierten Bilddaten vorgenommen wurde, da diese serverseitig unabhängig von der im Client-Gerät geladenen Bilddaten angewendet werden. Somit kann der Nutzer weiter an dem Client-Gerät arbeiten und Bearbeitungsbefehle erzeugen, die an das Serversystem gesendet werden, während Bilddaten von dem Serversystem nachgeladen werden. Dabei kann das Client-Gerät mittels einer bidirektionalen asynchronen Schnittstelle mit dem Serversystem kommunizieren, beispielsweise WebSockets. Durch die asynchrone Kommunikation bietet sich der Vorteil, dass der Nutzer keine Unterbrechung in seinem Arbeitsfluss hat und die neu zur Verfügung stehenden Bilddaten jederzeit von dem Serversystem an das Client-Gerät übertragen werden können abhängig lediglich von den Leistungsparametern.

Vorzugsweise umfasst das obige Verfahren ferner die folgenden Schritte: i) ein Visualisieren der bearbeiteten hochaufgelösten 3D-Bilddaten in dem Client-Gerät und j) Ersetzen eines Teils der Visualisierung der datenreduzierten Bilddaten mit der Visualisierung der hochaufgelösten 3D-Bilddaten. Sobald also neue Bilddaten an das Client-Gerät übertragen worden sind und dort visualisiert worden sind, kann die ursprüngliche Visualisierung durch eine Visualisierung der neu erlangten Bilddaten zumindest teilweise ersetzt werden. Dabei können die ursprünglich visualisierten Bilddaten Schrittweise durch die Visualisierung der neu erlangten Bilddaten ersetzt werden. Dies bietet den Vorteil, dass beispielsweise bei einer schlechten Internetverbindung und daher einer langsamen Datenübertragung vom Serversystem an das Client-Gerät, selbst wenige zur Verfügung stehende Bilddaten visualisiert werden und dem Nutzer direkt zur Verfügung gestellt werden können. Somit stehen dem Nutzer frühzeitig Informationen, die beispielsweise aus den Bearbeitungsbefehlen resultieren, zur Verfügung. Damit ist ein flüssiges Arbeiten selbst bei relativ schlechten Leistungsparametern des Client-Geräts möglich.

Vorzugsweise umfassen in Schritt e) die Bearbeitungsbefehle Informationen über einen Bereich von Interesse in der Visualisierung der datenreduzierten Bilddaten in dem Client-Gerät und in Schritt g) werden die hochaufgelösten 3D-Bilddaten in dem Bereich von Interesse als die bearbeiteten hochaufgelösten Bilddaten bereitgestellt. So kann beispielsweise ein Nutzer in der Visualisierung der datenreduzierten Bilddaten zu einer Position von Interesse navigieren und diese Position anschließend als Bereich von Interesse markieren, woraufhin lediglich dieser markierte Bereich von Interesse mit einer höheren Auflösung selektiv nachgeladen werden kann, wodurch der Bereich von Interesse detailreicher (d.h. hochaufgelöster) dargestellt werden kann. Mit anderen Worten können die datenreduzierten Bilddaten (bzw. deren Visualisierung) lediglich zur Orientierung und zur Navigation in dem gesamten medizinischen Bilddatensatz genutzt werden und basierend darauf ein Bereich von Interesse identifiziert und markiert werden, der dann mit einer höheren oder sogar mit der höchsten zur Verfügung stehenden Auflösung von dem Serversystem an das Client-Gerät übermittelt wird. Der Bereich von Interesse kann dann hochgenau analysiert und/oder untersucht werden. Dies bietet den Vorteil, dass ein hocheffizientes Arbeiten möglich ist, ohne den gesamten medizinischen Bilddatensatz laden zu müssen. Somit können auch leistungsschwächere Client-Geräte bei der Analyse von medizinischen Bilddaten effizient genutzt werden. Zudem ist es auch möglich die bessere Auflösung im Bereich von Interesse Stufenweise nachzuladen. Mit anderen Worten kann nach der Markierung des Bereichs von Interesse erst eine etwas bessere aber noch nicht die beste Auflösung von dem Serversystem an das Client-Gerät gesendet werden. Somit kann bewirkt werden, dass die hochaufgelösten Bilddaten einem Nutzer des Client-Geräts früher zur Verfügung stehen. Somit kann das Arbeiten noch weiter beschleunigt werden. Dazu kann der Nutzer eine Auswahl treffen, welche Auflösung der Bilddaten er als nächstes wünscht. Dadurch kann das Verfahren an die Bedürfnisse des Nutzers angepasst werden, um eine weitere Effizienzsteigerung zu bewirken. Schritt h) kann somit ein mehrfaches Übermitteln von den bearbeiteten Bilddaten von dem Serversystem an das Client-Gerät umfassen. Bei jedem Übermittlungsschritt kann die Auflösung der übermittelten Bilddaten gesteigert werden. Somit kann die Visualisierung in dem Client-Gerät ständig aktualisiert werden und der Nutzer erhält schnell Feedback von seinen Bearbeitungsbefehlen. Dieses schrittweise Nachladen kann solange laufen, bis die bearbeiteten Bilddaten in dem Client-Gerät dieselbe Auflösung wie die hochaufgelösten 3D-Bilddaten auf dem Serversystem haben oder bis ein Nutzer die schrittweise Übertragung stoppt, wenn für seine Bearbeitung beispielsweise eine ausreichende Auflösung vorliegt.

Vorzugsweise werden die Bearbeitungsbefehle durch einen Bedienkontext, insbesondere ein Verharren in einer Ansicht, ein Überfahren eines Bereichs in der Visualisierung der datenreduzierten Bilddaten mit der Maus ggf. mit gedrückter Maustaste, ein Doppelklick und/oder ein Klick auf eine Schaltfläche in dem Client-Gerät bestimmt. Mit anderen Worten können Bearbeitungsbefehle explizit durch einen Nutzer erzeugt werden oder basierend auf einer Interaktion (d.h. Bedienkontext) zwischen Nutzer und Client-Gerät implizit bestimmt werden. So kann beispielsweise ein Verharren in einer bestimmten Ansicht eines Nutzers dahingehend interpretiert werden, dass der Nutzer diesen Bereich genauer analysieren will und daher detailliertere Bilddaten für diesen speziellen Bereich wünscht. So können die Bearbeitungsbefehle eine implizite Bestimmung eines Bereichs von Interesse umfassen, der dann von dem Serversystem der Bearbeitung der hochaufgelösten Bilddaten zugrunde gelegt wird (Schritt g)), um die bearbeiteten Bilddaten zu erlangen, die dann wiederrum an das Client-Gerät übermittelt werden (siehe oben).

Für eine ärztliche Analyse kann eine Visualisierung der Bilddaten in der größtmöglichen Auflösung notwendig sein. Daher kann gemäß einem Aspekt der Erfindung über den Bedienkontext des Nutzers (Verharren in einer Ansicht, Doppelklick, Klick auf einen Button, etc.) erkannt werden, dass der aktuell visualisierte Bereich von besonderem Interesse ist. In diesem Fall kann eine Berechnung der bestmöglichen Qualität der Bilddaten in dem derzeit betrachteten Bereich auf Basis von an das Serversystem übermittelten Bearbeitungsbefehlen angestoßen werden. Parallel dazu kann über eine asynchrone Kommunikation serverseitig eine Visualisierung des Bereichs unter Verwendung der maximalen Auflösung veranlasst werden. Das Ergebnis wird je nach Geschwindigkeit der Datenverbindung zwischen Serversystem und Client-Gerät entweder vollständig oder zunächst in einer Zwischenauflösung an das Client-Gerät gesendet, wo es die derzeitige Visualisierung ersetzt. Somit kann die Visualisierung serverseitig vorgenommen werden, was bei leistungsschwachen Client-Geräten zu einem schnellen Ergebnis führt.

Vorzugsweise umfasst die Bearbeitung aus Schritt e) ein Zuschneiden und/oder eine Glättoperation und/oder eine Operation zur Vergrößerung, Verkleinerung oder Veränderung einzelner Bereiche der Visualisierung der datenreduzierten Bilddaten. Die Glättoperation sorgt dafür, dass Unebenheiten auf der Oberfläche eines Körpers reduziert werden. Operationen zum Vergrößern, Verkleinern und Verändern einzelner Bereiche können beispielsweise dazu verwendet werden, Oberflächenstruktur so zu verändern, dass Erhebungen vorgenommen werden oder beispielsweise einzelne Bereiche wie ein Zahn vergrößert werden können.

Vorzugsweise sind oder werden hochaufgelöste 3D-Bilddaten und die datenreduzierten Bilddaten demselben dreidimensionalen Koordinatensystem zugeordnet. Diese Zuordnung kann auf dem Serversystem durchgeführt werden, insbesondere in Schritt a). Durch eine solche Zuordnung ist es vorteilhaft möglich, Bilddaten mit unterschiedlichen Auflösungen in einer einzigen Visualisierung gleichzeitig darzustellen. Ferner kann die Visualisierung ohne Weiteres mit höher aufgelösten Bilddaten ergänzt werden. Dazu können alle Bilddaten (unabhängig von deren Auflösung) in einem Koordinatensystem dargestellt werden. Das Koordinatensystem hat vorzugsweise eine Schrittweite (Abstand zwischen Werten auf den Achsen, der auch als Raster bezeichnet werden kann), die der Auflösung der hochauflösenden 3D-Bilddaten entspricht. Mit anderen Worten kann das Koordinatensystem eine Schrittweite haben, die den am höchsten aufgelösten zur Verfügung stehenden Bilddaten entspricht. So ist es beispielsweise möglich, in einer Visualisierung von datenreduzierten Bilddaten einzelne Bereiche mit einer Visualisierung von hochaufgelösten Bilddaten zu ersetzen, wobei die Position der hochaufgelösten Bilddaten in dem Koordinatensystem eindeutig zugewiesen werden kann. Dabei kann ein dreidimensionales Koordinatensystem, welches als Einheit geometrische Längenmaße (wie beispielsweise Millimeter, Nanometer usw.) verwendet, genutzt werden. Dies ermöglicht auch die kombinierte Darstellung von Volumendaten und Geometriedaten auch dann, wenn die Daten aufgrund der Leistungsparameter zumindest anfangs nur in einer ungenaueren und gröberen Auflösung in dem Client-Gerät vorliegen. Weiterhin ermöglicht dies einzelne Datenbereiche selektiv nachzuladen, wenn der Nutzer beispielsweise in einen solchen Bereich hereinzoomt, um diesen Bereich detailgetreu in dem Client-Gerät zu visualisieren. Durch die detailgetreue Darstellung des Detailbereichs kann viel Speicherplatz und Downloadleistung gespart werden. Die hochaufgelösten medizinischen 3D-Bilddaten können Voxel umfassen, deren Position im Raum im Verhältnis zueinander bestimmt sind. Mit anderen Worten bestimmt ein Voxel seine Position im Raum basierend auf ein oder mehrere angrenzende(s) Voxel. Geometriedaten können dabei Polygone und/oder Punkte und/oder andere geometrische Objekte, z.B. Gitternetzmodelle, sein, die im Verhältnis zu einem Koordinatensystem definiert sind.

Mit anderen Worten wird bei den datenreduzierten Daten kein Koordinatensystem mit gröberem Raster angewendet, sondern ein Raster, das den hochaufgelösten 3D-Bilddaten entspricht (d.h. dem feinste zur Verfügung stehenden Raster). Dadurch ist gewährleistet, dass bei einer gemischten Darstellung von hochaufgelösten Bilddaten und datenreduzierten Bilddaten und/oder Geometriedaten alle Objekte in Bezug auf das Koordinatensystem mit dem feinsten Raster dargestellt sind. Dadurch können Verluste und/oder ungewollte Verschiebungen einzelner Objekte aufgrund eines gröberen Rasters vermieden werden.

Gemäß einem Aspekt der Erfindung werden sämtliche Visualisierungs- und auch Modifizierungsaktivitäten an das Serversystem übertragen, damit das Serversystem über die Nutzeraktionen informiert ist. Dies ermöglicht in Verbindung mit dem einheitlichen dreidimensionalen Koordinatensystem auf Basis von geometrischen Längen, bei einer schlechten Performance von Hardware bzw. schlechter Internetanbindung, serverseitig eine optimale Visualisierung auf Basis der hochaufgelösten 3D-Bilddaten zu erstellen und diese dem Client-Gerät zur Verfügung zu stellen, wo diese dann für eine Anzeige genutzt werden kann. Somit kann der Nutzer in einem vereinfachten 3D-Modell (d.h. die Visualisierung der datenreduzierten Bilddaten), welches schnell geladen werden kann, dynamisch an eine interessierende Stelle navigieren und dann trotz schlechter Hardware-Performance und Internetanbindung dennoch eine optimale Visualisierung in diesem Bereich erhalten.

Vorzugsweise wird parallel zu der Verarbeitung der hochaufgelösten 3D-Bilddaten in dem Serversystem in Schritt g) eine Bearbeitung der datenreduzierten Bilddaten in dem Client-Gerät durchgeführt.

Es kann beispielsweise der Fall auftreten, bei dem die Leistungsparameter (insbesondere die Internetverbindung) so schlecht ist, dass eine zügige Übermittlung von bearbeiteten Bilddaten an das Client-Gerät nicht möglich ist, selbst wenn diese so verarbeitet werden, dass sie ein geringeres Datenvolumen aufweisen. In einem solchen Fall können Bearbeitungsbefehle auch in dem Client-Gerät auf die dort vorhandenen datenreduzierten Bilddaten angewendet werden. Nichtsdestotrotz können die Bearbeitungsbefehle parallel dazu an das Serversystem gesendet werden und dort auf die hochaufgelösten Bilddaten angewendet werden, um bearbeitete Bilddaten zu erzeugen. Somit können sowohl in dem Client-Gerät als auch in dem Serversystem bearbeitete Bilddaten erlangt werden. Die bearbeiteten Bilddaten in dem Serversystem können abhängig von den Leistungsparametern (insbesondere abhängig von der Internetverbindung) an das Client-Gerät gesendet werden. Parallel dazu können die Bearbeitungsbefehle in dem Client-Gerät auf die datenreduzierten Bilddaten in dem Client-Gerät angewendet werden, um bearbeitete Bilddaten in dem Client-Gerät zu erlangen. Die Erzeugung von bearbeiteten Bilddaten in dem Client-Gerät kann abhängig von den Leistungsparametern (insbesondere von der Rechenleistung des Client-Geräts) sein. Es können dann die bearbeiteten Bilddaten für eine Visualisierung oder für eine Aktualisierung der Visualisierung genutzt werden, die zuerst in dem Client-Gerät vorliegen - entweder die vom Serversystem empfangenen oder die von dem Client-Gerät erzeugten. Somit kann sichergestellt sein, dass der Nutzer unter den vorhandenen Leistungsparametern schnellstmöglich eine Visualisierung (d.h. Feedback seiner Bearbeitung) erhält.

Vorzugsweise werden in Schritt c) zusätzlich Metadaten von dem Serversystem an das Client-Gerät übermittelt, wobei die Metadaten Informationen über die Datenreduzierten Bilddaten und/oder darüber umfassen, welche weiteren datenreduzierten Bilddaten auf dem Serversystem zur Verfügung stehen.

Mit anderen Worten kann das Client-Gerät Metadaten erhalten, die Informationen darüber umfassen, ob es sich um eine vereinfachte Version der hochaufgelösten medizinischen 3D-Bilddaten handelt und welche gröberen und feineren Auflösungen mit welchen Datenvolumina serverseitig angeboten werden. In Abhängigkeit der Leistungsparameter kann das Client-Gerät automatisch oder durch eine Nutzereingabe entscheiden, welche Daten asynchron nachgeladen werden. Insbesondere können dabei speicherärmere (d.h. datenreduzierte) Bilddaten vom Serversystem abgerufen werden, um eine Volumenvisualisierung bei geringer Performance der Grafikkarte in dem Client-Gerät während beispielsweise einer interaktiven Drehoperation des Nutzers anzuzeigen. Ferner können detailreichere Bilddaten von dem Serversystem asynchron nachgeladen werden, wenn beispielsweise die Internetverbindung langsam ist, die Hardware des Client-Gerät aber schnell genug zur Visualisierung von hochaufgelösten Bilddaten ist.

Vorzugsweise umfasst der Schritt a) eine Komprimierung und/oder eine Zerlegung von ursprünglichen Bilddaten und ein sequentielles Hochladen von den komprimierten und/oder zerlegten ursprünglichen Bilddaten auf das Serversystem mittels eines Uploaders, um die hochaufgelösten medizinischen 3D-Bilddaten auf dem Serversystem bereitzustellen.

Die ursprünglichen Bilddaten können die Bilddaten sein, die das System zur Erzeugung von 3D-Bilddaten ausgibt (Beispielsweise ein MRT, CT oder Ultraschallgerät). Die ursprünglichen Bilddaten zur Verwendung im Rahmen der Erfindung müssen von einem lokalen Datenträger oder dem bildgebenden Gerät auf das Serversystem hochgeladen werden. Aufgrund der Größe der Bilddaten ist ein Upload über eine klassische Browser-Funktion nachteilig, insbesondere in Hinblick auf Zeitbedarf und Reversionssicherheit. Vielmehr kann gemäß einem Aspekt der Erfindung ein plattformunabhängiges Upload-Programm genutzt werden, welches auf dem jeweiligen Rechner des Nutzers, der einen Upload plant, installiert werden kann. Das Upload-Programm kann über ein Verfahren zu Kompression und Zerlegung der ursprünglichen Bilddaten in kleine Teilmengen, die in mehreren parallel Datenverbindungen an das Serversystem übertragen werden, verfügen. Kommt es zu einer Unterbrechung der Internetverbindung, kann der Upload nahtlos an der Stelle fortgesetzt, wo er unterbrochen wurde.

Das Upload-Programm kann ferner Patientendaten, den medizinischen Verwendungszwecks und/oder die medizinischen Indikation erfassen. Die Identifikation des Patienten kann beispielsweise über eine digitale Signatur oder Hilfsweise anderer, eindeutig identifizierbarer Merkmale erfolgen. Ferner kann, insbesondere mittels künstlicher Intelligenz, unter Berücksichtigung aller zur Verfügung stehender Daten, aus den ursprünglichen Bilddaten lediglich jene Teile freigestellt werden, die im Hinblick auf die beabsichtigte Verwendung und/oder medizinische Indikation notwendig sind. Umfassen die ursprünglichen Bilddaten beispielsweise ein MRT eines Kiefers und soll der Patient ein Zahnimplantat bekommen, können die Bilddaten, die für einen Zahnarzt und Zahntechniker uninteressant sind, weggeschnitten werden. Somit können nicht benötigte Bereiche der ursprünglichen Bilddaten verworfen werden. Die Identifikation von benötigten Bereichen kann durch eine künstliche Intelligenz erfolgen. Dadurch kann erhebliches Datenvolumen eingespart werden, ohne dass eine manuelle Bearbeitung erforderlich ist.

Bei Initiierung eines Uploads kann eine eindeutige Transaktionsnummer generiert werden. Diese Transaktionsnummer kann dem Patienten übergeben werden sowie zur Dokumentation und/oder Nachweis gespeichert werden. Der Uploader kann also eine interoperable Schnittstelle zur Anbindung beliebiger Drittsysteme im Intranet bereit bereitstellen. Über diese Schnittstellen können die kompletten Funktionalitäten des Uploaders genutzt werden. Dadurch ist gewährleistet, dass die bildgebenden Geräte ohne zusätzliche Nutzerinteraktion automatisch die ursprünglichen Bilddaten an das Serversystem übermitteln können. Somit können die hochaufgelösten 3D-Bilddaten einfach und sicher auf dem Serversystem bereitgestellt werden.

Vorzugsweise umfasst das Verfahren vor Schritt c) ferner die folgenden Schritte: Bestimmen von Patienteninformationen auf Basis der hochaufgelösten 3D-Bilddaten, insbesondere mittels künstlicher Intelligenz, Vergleichen von den bestimmten Patienteninformationen mit den tatsächlichen Patienteninformationen, und Ausgeben einer Warnung, wenn die bestimmten Patienteninformationen nicht mit den tatsächlichen Patienteninformationen übereinstimmen.

Mit anderen Worten kann eine künstliche Intelligenz (beispielsweise ein künstliches neuronales Netzwerk) eingesetzt werden, das trainiert ist, basierend auf Bilddaten, insbesondere auf Volumendaten von menschlichen Gebissen, das Alter, Geschlecht und weitere Metadaten zum Patienten zu erkennen. So kann die künstliche Intelligenz beispielsweise eingesetzt werden, um die Metadaten der hochaufgelösten medizinischen 3D-Bilddaten auf dem Serversystem zu bestimmen. Demnach kann anhand des Gebisses auf das Alter und auf weitere Daten des Patienten geschlossen werden. Die so bestimmten Metadaten des Patienten werden mit den tatsächlichen Metadaten des Patienten verglichen, die beispielsweise als zusätzliche Informationen auf dem Serversystem abgelegt sind. Ferner könne die tatsächlichen Metadaten aus einer elektronischen Patientenakte entnommen oder dem Serversystem auf eine andere Weise zur Verfügung gestellt werden. Weichen die Metadaten, die von der künstlichen Intelligenz bestimmt wurden, von den tatsächlichen Metadaten ab, wird eine Warnung an den Nutzer ausgegeben. So kann verhindert werden, dass beispielsweise veraltete hochaufgelöste medizinische 3D-Bilddaten auf dem Serversystem einer weiteren Bearbeitung zugrunde gelegt werden. Ferner können so Verwechslungen zwischen Patienten vermieden werden. Das Bestimmen der Metadaten kann bei dem obigen Verfahren auch ohne das anschließende Vergleichen mit den tatsächlichen Metadaten erfolgen, wenn beispielsweise keine Metadaten von dem Patienten vorliegen. Somit kann der Nutzer, der die hochaufgelösten 3D-Bilddaten an dem Client-Gerät bearbeitet, mehr Informationen nutzen, die eine genauere Analyse und/oder Bearbeitung der Bilddaten ermöglichen kann. Beispielsweise in dem Fall, bei dem der Nutzer ein Zahntechniker ist, kann er somit in der Lage sein, bei der Auswahl von Zahnkronen auf weitere Informationen des Patienten zurückzugreifen, wodurch leichter eine passende Zahnkrone für den Patienten gewählt werden kann.

Vorzugsweise weist das Verfahren ferner die folgenden Schritte auf: Bereitstellen einer Visualisierung der hochaufgelösten 3D-Bilddaten in dem Serversystem, insbesondere basierend auf Metadaten der hochaufgelösten 3D-Bilddaten und Übermitteln der Visualisierung der hochaufgelösten 3D-Bilddaten von dem Server an das Client-Gerät. Dabei kann beispielsweise eine künstliche Intelligent eingesetzt werden, die trainiert ist in hochaufgelösten 3D-Bilddaten abgebildete Objekte, wie Körperregionen, Knochen und Organe zu erkennen. Für jedes dieser Objekte kann eine bevorzugte Visualisierungseinstellung aus dem Bedienverhalten der Nutzer erlernt sein. Visualisierungseinstellung können dabei bestimmte Ansichten (2D oder 3D) sein, die insbesondere in der Zahnmedizin verwendet werden. Beispielsweise kann eine Visualisierungseinstellung die Freistellung der Zähne sein. Zusätzlich oder alternativ kann eine Vorderansicht der Zähne abgebildet werden. Darüber hinaus kann die Visualisierungseinstellung auch bestimmte Schnitte durch die Bilddaten (z.B. ein MPR Schnitt durch eine bestimmte Schicht) sein. die Des Weiteren kann im Hinblick auf die zu den hochaufgelösten 3D-Bilddaten auf dem Serversystem erfassten Metadaten eine Priorisierung der in dem jeweiligen Datensatz entdeckten Betrachtungsobjekte errechnet werden. Entsprechend dieser Priorisierung kann dem Nutzer eine direkte Navigation zu den relevantesten Betrachtungsobjekten angeboten werden. Beispielsweise könnten dies ein freigestellter Unterkiefer, der von oben abgebildet ist, sein. Ferner kann dies ein freigestellter Oberkiefer sein, der von unten dargestellt ist. Ferner ist denkbar so alle Zähne einzeln oder als gesamtes darzustellen oder das Zahnfleisch. Somit bietet sich der Vorteil, dass ein Nutzer direkt eine zu bevorzugende Ansicht wählen kann, die dann von dem Serversystem als Visualisierung direkt an das Client-Gerät in einer besseren bzw. detailgetreueren Auflösung übermittelt werden kann, verglichen mit dem Fall, bei dem zuerst die gesamten Bilddaten als datenreduzierte Bilddaten zur Verfügung gestellt werden müssen, damit der Nutzer einen gewünschten Bereich identifizieren kann. Ferner kann durch die Erzeugung der Visualisierung in dem Serversystem das Client-Gerät eine geringe Performance aufweisen, da die Bilddaten nicht in dem Clientgerät visualisiert werden müssen, sondern schon als Visualisierung von dem Serversystem zur Verfügung gestellt werden. Es ist denkbar, dass der Nutzer eine Übersicht über die zur Verfügung stehenden Visualisierungen auf dem Serversystem erhält und daraus die gewünschte auswählen kann. Nach erfolgter Auswahl, kann diese Visualisierung abhängig von den Leistungsparametern vollständig oder in mehreren Schritten von dem Serversystem an das Client-Gerät übermittelt werden.

Medizinische Bilddaten können von unterschiedlichen Stellen eines menschlichen Körpers generiert werden. Je nach medizinischer Indikation sind unterschiedliche Stellen für einen Nutzer von Interesse. Daher kann es für den Nutzer von Interesse sein nur eine Stelle zu visualisieren, um diese untersuchen zu können. Gemäß einem weiteren Aspekt der Erfindung können übliche Visualisierungen von Körperregionen erlernt und erkannt werden. Insbesondere basierend auf einer Fachrichtung des Nutzers, der medizinischen Indikation und/oder den abgebildeten Bereichen des Körpers kann eine künstliche Intelligenz trainiert werden, welche Visualisierungen (Ebene, Winkel, Vergrößerung, Filterung hinsichtlich Dichte, etc.) relevant sein können. Diese Visualisierungen können dann direkt beim Bereitstellen der Daten (Schritt a)) erstellt und dem Nutzer zusätzlich zur interaktiven Visualisierung der datenreduzierten Bilddaten ohne Berechnungslatenz zur Verfügung gestellt werden. Dadurch kann eine schnelle und einfache Handhabung für den Nutzer bereitgestellt werden.

Gemäß einem weiteren Aspekt der vorliegenden Erfindung wird das obige Verfahren in der Zahnmedizin verwendet, wobei die hochaufgelösten 3D-Bilddaten in dem Serversystem Bilddaten eines menschlichen Kiefers umfassen.

Besonders im zahnmedizinischen Bereich werden Bilddaten an vielen verschiedenen Stellen (und damit auch unterschiedlichen Orten) verwendet. Beispielsweise werden die Bilddaten durch einen Arzt zur Analyse der Ist-Situation herangezogen. Ferner werden in der Kieferchirurgie die Bilddaten verwendet, um etwaige chirurgische Eingriffe zu planen und/oder zu simulieren. Ferner gibt es externe Zulieferer wie beispielsweise ein medizintechnisches Labor, das Implantate und ähnliche Instrumente zur Anbringung in dem menschlichen Kiefer individuell plant und herstellt. Das heißt die medizinischen Bilddaten werden von vielen verschiedenen und unabhängig arbeitenden Stellen verwendet. Daher ist im zahnmedizinischen Bereich eine einfache, schnelle, effiziente und datenrechtskonforme Verwaltung und Verteilung von Bilddaten von Nöten. Das obige Verfahren erfüllt diese Aufgaben mit den obigen Merkmalen und Vorteilen.

Gemäß einem weiteren Aspekt der vorliegenden Erfindung wird ein Computerprogramm bereitgestellt, das Befehle umfasst, die, wenn das Programm auf einem oder mehreren Prozessoren ausgeführt wird, den oder die Prozessoren veranlassen, das obige Verfahren auszuführen. Das Computerprogramm kann in den internen Speicher eines digitalen Computers ladbar sein und Softwarecodeabschnitte zur Durchführung des erfindungsgemäßen Verfahrens umfassen, wenn das Produkt auf einer Recheneinheit ausgeführt wird. Auch wenn gemäß einem Aspekt der vorliegenden Erfindung keine Installation einer Software (abgesehen von einer allgemeinen Browsersoftware) notwendig ist, kann dennoch Programmcode auf das Client-Gerät geladen werden der in dem Speicher des Browsers ausgeführt wird. Die Erfindung ist ferner auf ein nicht-transitorisches computerlesbares Medium gerichtet, das einen Satz von Anweisungen umfasst, die, wenn sie von einem Prozessor ausgeführt werden, dazu führen, dass eine Reihe von Schritten durchgeführt wird, die das erfindungsgemäße Verfahren ausführen. Die Erfindung kann ferner in einem greifbaren computerlesbaren Speichermedium implementiert sein, das einen Computerprogrammcode enthält, der von einem Prozessor auszuführen ist, wobei der Computerprogrammcode, wenn er ausgeführt wird, das erfindungsgemäße Verfahren implementiert.

Gemäß einem weiteren Aspekt der vorliegenden Erfindung wird ein System zur Analyse von medizinischen 3D-Bilddaten in einem Client-Gerät bereitgestellt, wobei das System umfasst: Ein Serversystem mit einer ersten Recheneinheit, ein Client-Gerät, das eine zweite Recheneinheit aufweist und mit dem Serversystem verbunden oder verbindbar ist, wobei die erste Recheneinheit und die zweite Recheneinheit dazu ausgestaltet sind das obige Verfahren auszuführen.

Alle Vorteile und Weiterbildungen des Verfahrens gelten analog auch für die Vorrichtung. Merkmale einzelner Ausführungsformen können mit Merkmalen anderer Ausführungsformen kombiniert werden und weisen die den Merkmalen zugewiesenen Vorteile und Wirkungen auf.

Zu bevorzugende Ausführungsformen der Erfindung werden nun mit Bezug auf die beigefügten Zeichnungen beschrieben, in denen
- **Fig. 1**: eine schematische Ansicht eines Systems zur Analyse von medizinischen 3D-Bilddaten gemäß einer Ausführungsform der vorliegenden Erfindung zeigt,
- **Fig. 2**: ein Ablaufdiagram eines Verfahrens zur Analyse von medizinischen Bilddaten gemäß einer Ausführungsform der vorliegenden Erfindung zeigt, und
- **Fig. 3**: ein Ablaufdiagram weiterer Schritte eines Verfahrens zur Analyse von medizinischen Bilddaten gemäß einer weiteren Ausführungsform der vorliegenden Erfindung zeigt.

Ausführungsformen der vorliegenden Erfindung liefern eine Webanwendung, die die Visualisierung von medizinischen Volumendaten (Voxel) ermöglicht, die üblicherweise als Ergebnis von Computertomographie (CT) oder Magnet Resonanz Tomographie (MRT) generiert werden. Da die Größe des Datensatzes mit mehreren hundert Megabyte zu groß ist, um diesen in den Speicher eines Browsers zu laden, wird dieser dynamisch unter Verwendung von technischen Parametern des Client-Gerätes des Nutzers (wie Prozessorgeschwindigkeit, Internetanbindung, Auflösung des Bildschirms) serverseitig heruntergerechnet und anschließend übertragen. Dadurch wird es ermöglicht, auf die jeweilige Leistungsfähigkeit des Endgeräts ausgerichtete Betrachtungsmöglichkeiten in einer Webanwendung zu realisieren.

Für die interaktive Betrachtung medizinischer Volumendaten wird dem Nutzer einer Webanwendung ermöglicht, mit dem auf den jeweiligen Geräten zur Verfügung stehenden Bedienelementen eine zweidimensionale Ebene in den dreidimensionalen Datenpunkten zu visualisieren. Diese dreidimensionale Navigation und Visualisierung ist sehr rechenintensiv, insbesondere wenn diese Rechenoperationen im Browser auf einer leistungsschwachen Hardware durchgeführt werden. Insofern wird seitens der Anwendung ein stark vereinfachtes Datenmodell bereitgestellt, welches für Visualisierungszwecken während der Navigation verwendet wird, um beispielsweise flüssig den im MRT abgebildeten Körper von allen Seiten betrachten zu können.

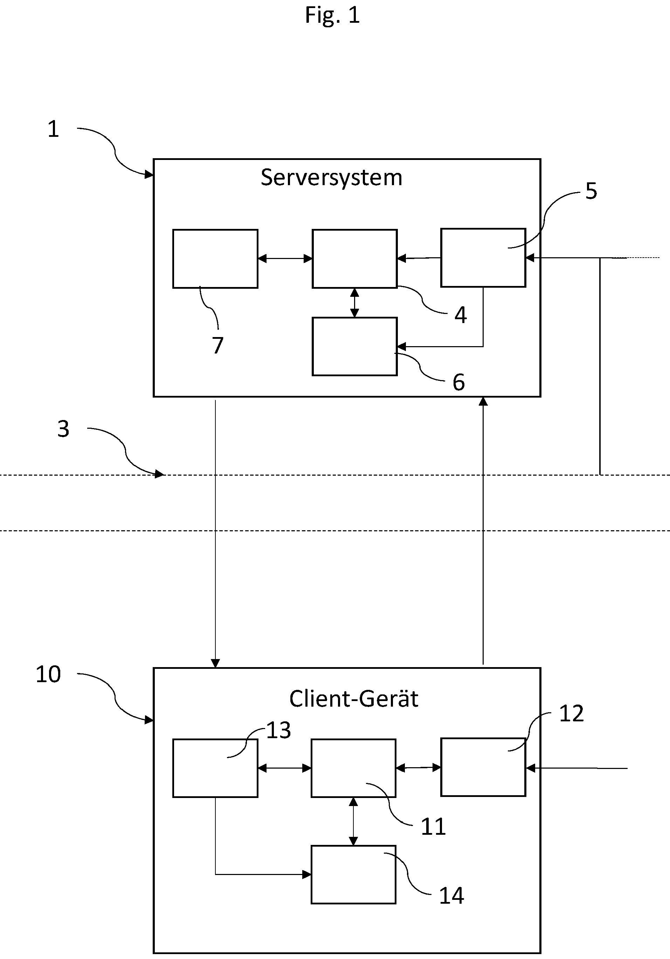

In Fig. 1 ist eine allgemeine Systemübersicht gemäß einer Ausführungsform der vorliegenden Erfindung dargestellt. Die allgemeine Systemübersicht in Fig. 1 zeigt ein clientseitiges Client-Gerät 10 (unterer Bereich in Fig. 1) und ein serverseitiges Serversystem 1 (oberer Bereich in Fig. 1). Das Serversystem 1 und das Clientgerät 10 sind durch ein Netzwerk, wie beispielsweise dem Internet 3, miteinander verbunden oder verbindbar. Das Serversystem 1 umfasst eine erste Recheneinheit 4, die dazu ausgestaltet ist, hochaufgelöste medizinische 3D-Bilddaten zu verarbeiten, um datenreduzierte Bilddaten zu erzeugen. Ferner umfasst das Serversystem 1 eine Schnittstelle 5, über die die hochaufgelösten medizinischen 3D-Bilddaten einem in dem Serversystem 1 vorhandenen Speichersystem 6 und/oder der ersten Recheneinheit 4 zugeführt werden können. Die Schnittstelle 5 ist über einen nicht dargestellten Uploader mit einer Datenbank (beispielsweise eines PAC-Systems eines Krankenhauses) verbunden oder verbindbar, um die hochaufgelösten medizinischen 3D-Bilddaten zu empfangen. Beispielsweise ist die Schnittstelle 5 über das Internet mit einer solchen Datenbank verbunden oder verbindbar. Ferner umfasst das Serversystem 1 ein Server-Sende-Empfangseinheit 7, die dazu ausgestaltet ist, Daten von dem Serversystem 1 an das Client-Gerät 10 zu übertragen und von diesem zu empfangen.

Das Client-Gerät 10 umfasst eine Client-Sende-Empfangseinheit 13, die dazu ausgestaltet ist, Daten von dem Serversystem 1 zu empfangen und an dieses zu senden. Ferner umfasst das Client-Gerät 10 eine zweite Recheneinheit 11, die dazu ausgestaltet ist, Bilddaten zu verarbeiten. Insbesondere ist die zweite Recheneinheit 11 dazu ausgestaltet, Bilddaten zu visualisieren und Bilddaten zu bearbeiten beispielsweise zuzuschneiden und/oder zu glätten. Die von dem Serversystem 1 empfangenen Bilddaten können in einer Client-Gerät-Speichereinheit 14 gespeichert werden. Bilddaten können in dem Client-Gerät 10 durch die zweite Recheneinheit 11 visualisiert werden und über eine Client-Schnittstelle 12 einem Nutzer dargestellt werden. Ferner kann der Nutzer über die Client-Schnittstelle 12 die Visualisierung der Bilddaten bearbeiten, um Bearbeitungsbefehle zu erzeugen. Die Bearbeitungsbefehle können dann von der Client-Sende-Empfangseinheit 13 an das Serversystem 1 übermittelt werden. Ferner können die Bearbeitungsbefehle durch die zweite Recheneinheit 11 auf die Bilddaten in dem Client-Gerät 10 angewendet werden. Die erste Recheneinheit 4 ist leistungsstärker als die zweite Recheneinheit 11.

Die zweite Recheneinheit 11 ist dazu ausgestaltet, zu Beginn einer Bearbeitung durch einen Nutzer Leistungsparameter des Client-Geräts 10 mittels der Client-Sende-Empfangseinheit 13 an das Serversystem 1 zu übermitteln. Die Leistungsparameter sind vorzugsweise die Upload- und Downloadgeschwindigkeit, mit der das Client-Gerät 10 an das Internet 3 angeschlossen ist und/oder die Hardware Performance des Client-Geräts 10, wie beispielsweise die Rechenleistung der zweiten Recheneinheit 11, Visualisierungsleistung, Grafikkarte usw.

Die erste Recheneinheit 4 in dem Serversystem 1 ist dazu ausgestaltet, basierend auf den von dem Client-Gerät 10 empfangenen Leistungsparametern, die hochaufgelösten medizinischen 3D-Bilddaten so zu verarbeiten, dass datenreduzierte Bilddaten erlangt werden. Diese datenreduzierten Bilddaten werden dann in der Speichereinheit 6 des Serversystems 1 zwischengespeichert. Die Server-Sende-Empfangseinheit 7, des Serversystems 1 übermittelt anschließend die datenreduzierten Bilddaten an das Client-Gerät 10. Da die datenreduzierten Bilddaten ein Datenvolumen aufweisen, das an die jeweiligen Randbedingungen (Internetgeschwindigkeit und Performanceparameter des Client-Geräts 10, d.h. an die Leistungsparameter) angepasst ist, kann diese Übertragung von dem Serversystem 1 auf das Client-Gerät 10 schnell erfolgen. Im Client-Gerät 10 werden die datenreduzierten Bilddaten durch die zweite Recheneinheit 11 visualisiert und über die Schnittstelle 12 dem Nutzer dargestellt. Somit hat der Nutzer schnellstmöglich eine erste Visualisierung der datenreduzierten Bilddaten, um mit der Analyse der medizinischen Bilddaten zu beginnen.

In einer bevorzugten Ausführungsform der vorliegenden Erfindung erfolgt parallel zu der Analyse der ersten zur Verfügung gestellten Visualisierung der datenreduzierten Bilddaten ein selektives Nachladen von weiteren datenreduzierten Bilddaten, die eine höhere Auflösung aufweisen, als die zuerst erlangten datenreduzierten Bilddaten. Dazu kann die Client-Sende-Empfangseinheit 13 des Client-Geräts 10 dazu ausgestaltet sein, automatisch Informationen über den von dem Nutzer betrachteten Bereich an das Serversystem 1 zu übermitteln, woraufhin das Serversystem 1 in genau diesem Bereich höher aufgelöste aber noch nicht maximal aufgelöste Bilddaten zur Verfügung stellt und an das Client-Gerät 10 übermittelt. Sobald Bilddaten mit einer höheren Auflösung in dem Client-Gerät 10 durch die Client-Sende-Empfangseinheit 13 empfangen werden, visualisiert die zweite Steuereinheit 11 diese neu empfangenen Bilddaten und ersetzt die ursprüngliche Visualisierung 12 teilweise durch eine Visualisierung der neuen Bilddaten. Mit anderen Worten werden parallel zu der Bearbeitung durch den Nutzer im Hintergrund Bilddaten nachgeladen, die mehr Informationen umfassen (das heißt eine höhere Auflösung haben) als die ursprünglich geladenen Bilddaten. Während auf diese Weise dem Client-Gerät 10 weitere Bilddaten zur Verfügung gestellt werden (das Client Gerät 10 also Daten empfängt) sendet das Client-Gerät 10 weiterhin Informationen über das Nutzerverhalten (Bearbeitungsbefehle) an das Serversystem 1. Ferner werden auch die Leistungsparameter laufend aktualisiert. So kann beispielsweise bei nur einer temporär schlechten Internetverbindung sichergestellt sein, dass bei einer besseren zur Verfügung stehenden Bandbreite automatisch höher aufgelöste Bilddaten von dem Serversystem 1 an das Client-Gerät 10 übertragen werden können.

Ferner kann der Nutzer über die Schnittstelle 12 die Visualisierung der datenreduzierten Bilddaten aktiv bearbeiten um Bearbeitungsbefehle zu erzeugen, die auf die datenreduzierten Bilddaten in dem Client-Gerät angewendet werden können und/oder an das Serversystem 1 übermittelt werden können. Im Unterschied zu den zuvor implizit bestimmten Informationen über einen Bereich von Interesse durch den Bedienkontext des Nutzers, werden hier durch eine aktive Steuerung des Nutzers die Befehlsinformationen des Nutzers erzeugt. Mit anderen Worten können in dem Client-Gerät 10 auf zweierlei Weise Befehlsinformationen erzeugt werden, nämlich durch eine Analyse der von dem Nutzer durchgeführten Bearbeitung der Visualisierung (d.h. implizit durch den Nutzer vorgegeben) und durch eine aktive Bearbeitung der Visualisierung durch den Nutzer (d.h. durch eine direkte Befehlseingabe von dem Nutzer).

Der Nutzer kann beispielsweise die Visualisierung der datenreduzierten Bilddaten glätten, wegschneiden oder sonstige Analysefunktionen durchführen, wodurch Bearbeitungsbefehle erzeugt werden, die durch die Client-Sende-Empfangseinheit 13 von dem Client-Gerät 10 an das Serversystem 1 übermittelt werden. In dem Serversystem 1 werden die Bearbeitungsbefehle, die von dem Client-Gerät 10 aufgenommen werden, dann durch die erste Steuereinheit 4 auf die hochaufgelösten 3D-Bilddaten angewendet. Die so bearbeiteten hochaufgelösten 3D-Bilddaten werden in dem Speicher 6 des Serversystems 1 gespeichert und basierend auf den Leistungsparametern an das Client-Gerät 10 parallel zu der Bearbeitung der Visualisierung in dem Client-Gerät 10 durch den Nutzer übermittelt. Ferner werden auch die Bearbeitungsbefehle in dem Speicher des Serversystems 1 gespeichert. Sobald in dem Client-Gerät 10 neue Bilddaten zur Verfügung stehen, visualisiert die zweite Recheneinheit 11 diese und ersetzt die vorherige Visualisierung entsprechend. Damit ist ein kontinuierliches Arbeiten möglich, bei dem permanent im Hintergrund in Abhängigkeit der Leistungsparameter besser aufgelöste und/oder bearbeitete Bilddaten nachgeladen werden.

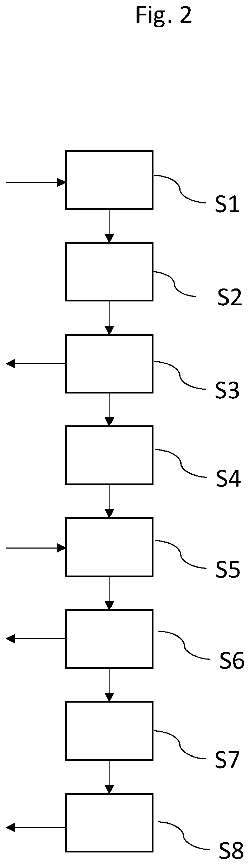

Figur 2 ist ein schematisches Ablaufdiagram, das ein Verfahren zur Analyse von medizinischen Bilddaten gemäß einer Ausführungsform der vorliegenden Erfindung darstellt. Die horizontalen Pfeile verdeutlichen in welchem Schritt Informationen und/oder Daten abgeben (Pfeil in Fig. 2 nach links) oder aufgenommen (Pfeil in Fig. 2 nach rechts) werden. In Schritt S1 werden hochaufgelöste medizinische 3D-Bilddaten auf einem Serversystem 1 bereitgestellt. Dazu werden die hochaufgelösten 3D-Bilddaten beispielsweise durch einen Uploader auf das Serversystem 1 hochgeladen. Dabei handelt es sich bei den hochaufgelösten medizinischen 3D-Bilddaten um Volumendaten, die beispielsweise durch ein MRT-Gerät oder CT-Gerät erzeugt wurden. Bei dem Hochladen werden die hochaufgelösten 3D-Bilddaten in kleine Teilmengen komprimiert und zerlegt, die in mehreren parallelen Datenverbindungen an das Serversystem 1 übertragen werden. Dies bietet den Vorteil, dass es im Falle einer Unterbrechung der Verbindung zwischen dem Uploader und dem Serversystem 1, der Upload nahtlos an der Stelle, an der er unterbrochen wurde, fortgesetzt werden kann. Der Uploader kann beispielsweise eine Instanz umfassen, die basierend auf den Metadaten automatisch die hochaufgelösten 3D-Bilddaten in einem Bereich von Interesse freistellt, sodass die medizinischen 3D-Bilddaten den beabsichtigten Verwendungszweck bzw. den medizinischen Indikationen entsprechen. So kann beispielsweise bei einer Analyse von einem menschlichen Kiefer dieser automatisch durch den Uploader freigestellt werden, sodass die auf das Serversystem 1 zu übertragene Datenmenge deutlich reduziert sein kann.

In Schritt S2 werden die hochaufgelösten 3D-Bilddaten in Abhängigkeit der Leistungsparameter des Client-Geräts 10 verarbeitet, um datenreduzierten Bilddaten zu erlangen. Dabei kann beispielsweise die Dichte der hochaufgelösten 3D-Bilddaten verringert werden, um die datenreduzierten Bilddaten zu erlangen. So kann beispielsweise jedem Bildpunkt (Voxel) eine eindeutige Koordinate zugeordnet weren, die die Position in einem Koordinatensystem definiert. Ferner kann dem Bildpunkt noch weitere Information, wie zum Beispiel ein Farbwert zugeordnet sein.

In Schritt S3 werden die datenreduzierten Bilddaten an das Client-Gerät 10 übermittelt. In dem Client-Gerät 10 werden die datenreduzierten Bilddaten durch die Client-Sende-Empfangseinheit 13 aufgenommen. Da die datenreduzierten Bilddaten auf Basis der Leistungsparameter erzeugt wurden, erfolgt die Übermittlung von dem Serversystem 1 zu dem Client-Gerät sehr zügig.

Anschließend wird in Schritt S4 eine Visualisierung der datenreduzierten Bilddaten erzeugt, und über die Schnittstelle 12 einem Nutzer dargestellt. Durch die Schnittstelle 12 ist dem Nutzer in Schritt S5 erlaubt, die Visualisierung der Datenreduzierten Bilddaten zu bearbeiten, um Bearbeitungsbefehle zu erzeugen. In Schritt S6 werden die Bearbeitungsbefehle von dem Client-Gerät 10 an das Serversystem 1 übermittelt. Dabei kann beispielsweise eine Information über einen Standort (in Bezug auf das eindeutige Koordinatensystem) und eine Art der Bearbeitung umfassen. Die so an das Serversystem 1 übermittelten Bearbeitungsbefehle werden dann auf die hochaufgelösten 3D-Bilddaten angewendet, um bearbeitete hochaufgelöste 3D-Bilddaten zu erlagen (S7). Auf dem Serversystem 1 werden dann die entsprechenden Bearbeitungsbefehle durch eine auf dem Serversystem 1 vorgesehen Software ausgeführt. In Schritt S8 werden dann die bearbeiteten hochaufgelösten 3D-Bilddaten von dem Serversystem an das Client-Gerät 10 übertagen.

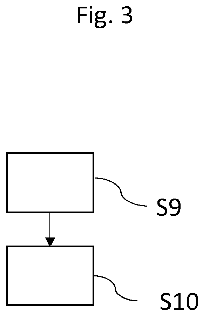

In Figur 3 sind weitere Verfahrensschritte des obigen Verfahrens schematisch dargestellt. Demgemäß können in Schritt S9 die bearbeiteten hochaufgelösten 3D-Bilddaten, die an das Client-Gerät 10 übertragen worden sind, dort visualisiert werden und entsprechende Teile in Schritt S10 der bereits in dem Client-Gerät 10 vorgesehenen Visualisierung der datenreduzierten Bilddaten ersetzten. Dadurch kann eine Visualisierung erzeugt werden, die sowohl auf datenreduzierten Bilddaten und auch höher aufgelösten Bilddaten basiert.

Ferner kann das erfindungsgemäße Verfahren in der Zahnmedizin verwendet werden und um weitere Schritte zur Modellierung und/oder Auswahl von Zahnimplantate umfassen. Zahnimplantate stellen künstliche Zahnwurzeln dar und werden im Knochen des Patienten verankert. Ein Zahntechniker erstellt eine Bohrschablone unter Zuhilfenahme von Volumendaten (meist ein MRT-Bild) und stellt bei der Modellierung sicher, dass beispielsweise keine Nerven durch das Implantat verletzt werden oder Winkel und Position des Implantats so angeordnet werden, dass sie mit Bohrern durch den geöffneten Mund eingeschraubt werden können. Auf das Implantat wird ein sogenanntes Abutment gesetzt, welches das Implantat mit der Zahnkrone verbindet. Diese Abutments können abgewinkelt sein, damit die Krone in die Reihe der Bestandszähne passt, obwohl das Implantat aufgrund der räumlichen Situation einen anderen Winkel aufweist. Die Ausrichtung des Implantats und die Auswahl des Abutments ist eine herausfordernde Arbeit für den Zahntechniker, die viel Erfahrung und Produktkenntnis erfordert und im Fall einer Fehlplanung zu ästhetischen und gesundheitlichen Schäden beim Patienten führen kann. Daher kann das obige Verfahren in einer Ausführungsform um eine künstliche Intelligenz erweitert werden, die den Zahntechniker bei der Modellierung von Implantat und Abutments unterstützt. Aufgrund der erlernten Daten erfolgt eine Bestimmung eines Vorschlags für Implantat und Abutment. Modifiziert der Nutzer diesen Vorschlag oder modelliert er von Hand, unterstützt die künstliche Intelligenz, indem sie Nebenbedingung überprüft und bei Fehlern warnt, etwa wenn ein gewinkeltes Abutment aufgrund des zur Verfügung stehenden Platzes zwischen den Zähnen nicht mehr gedreht und somit verschraubt werden könnte.

Zahnkronen werden von diversen Herstellern in unterschiedlichen Varianten angeboten, die sich insbesondere hinsichtlich Farbe, Größe und Form unterscheiden. Es ist die Aufgabe von Zahnarzt und Zahntechniker die Produkte so auszuwählen, dass die künstlichen Zähne den Originalzähnen möglichst ähnlich sind - also Form, Farbe und Größe den Originalzähnen entsprechen. Darüber hinaus variieren die Kosten der Zahnkronen, so dass die Zahnungsbereitschaft des Kunden ein weiteres Auswahlkriterium bei der Produktauswahl darstellt. Das Ergebnis der Produktauswahl ist somit aktuell von den individuellen persönlichen Fähigkeiten und des Kenntnisstandes hinsichtlich der Produkte des Zahnarztes oder Zahntechnikers abhängig. Das obige Verfahren verwendet eine künstliche Intelligenz, die Form und Größe der vorhandenen Zähne anhand der hochaufgelösten 3D-Biddaten erkennt. Liegen weitere Daten vor, wie ein Foto der Originalzähle oder ein bestimmter Farbton der Originalzähne, werden diese ebenfalls berücksichtigt. Aus diesen Daten wird ein Modell generiert, welches an eine künstliche Intelligenz übergeben wird. Diese analysiert anonymisiert sämtliche vorhandenen und zugänglichen Gebiss und Zahnersatzdaten, um für die künstlich nachzubildenden Zähne ein dem Original möglichst nahekommendes Produkt zu identifizieren. Die Ergebnisse werden so aufbereitet, dass der Patient das Ergebnis nach seinen Präferenzen selektieren kann, indem er beispielsweise den Preis oder Produkteigenschaften festlegt. Für jede Produktalternative kann die künstliche Intelligenz eine realitätsnahe visuelle Simulation zur Veranschaulichung des optischen Eindrucks der Implantate im Gebiss des Patienten bestimmen. Hierzu werden die dreidimensionalen Oberflächenstrukturen der Produkte (Geometriedaten) mit den 3D-Bilddaten des Patienten, insbesondere Daten über das sichtbare Zahnfleisch sowie etwaig vorhandenes Fotomaterial, verwendet. Die künstliche Intelligenz erkennt insbesondere die zu ersetzenden Originalzähne bzw. vorhandene Zahnlücken in vorhandenem Fotomaterial uns setzt passende Zahnkronen ein. Die Visualisierung passt automatisch die Größenproportionen, Belichtung und Position an die Gegebenheiten an.

## Patentansprüche

1. Computerimplementiertes Verfahren zur Analyse von medizinischen Bilddaten mit einer webbasierten Anwendung auf einem Client-Gerät (10), wobei das Verfahren umfasst:
a) Bereitstellen von hochaufgelösten medizinischen 3D-Bilddaten auf einem Serversystem (1),
b) Verarbeiten der hochaufgelösten 3D-Bilddaten in Abhängigkeit von Leistungsparametern des Client-Geräts (10), um datenreduzierte Bilddaten zu erlangen,
c) Übermitteln der datenreduzierten Bilddaten an das Client-Gerät (10),
d) Erzeugen einer Visualisierung der datenreduzierten Bilddaten in dem Client-Gerät (10),
e) Erlauben einer Bearbeitung der Visualisierung der datenreduzierten Bilddaten in dem Client-Gerät (10) durch einen Nutzer, um Bearbeitungsbefehle zu erzeugen,
f) Übermitteln der Bearbeitungsbefehle von dem Client-Gerät (10) an das Serversystem (1),
g) Anwenden der Bearbeitungsbefehle auf die hochaufgelösten 3D-Bilddaten, um bearbeitete Bilddaten zu erlangen,
h) Übermitteln zumindest eines Teils der bearbeiteten Bilddaten von dem Serversystem (1) an das Client-Gerät (10).

2. Verfahren gemäß Anspruch 1, wobei in Schritt h) zumindest ein Teil der bearbeiteten Bilddaten von dem Serversystem (1) per asynchroner Kommunikation an das Client-Gerät (10) übermittelt wird.

3. Verfahren gemäß einem der vorhergehenden Ansprüche ferner umfassend:
i) Visualisieren der bearbeiteten Bilddaten in dem Client-Gerät (10), und
j) Ersetzen eines Teils der Visualisierung der datenreduzierten Bilddaten mit der Visualisierung der bearbeiteten Bilddaten.

4. Verfahren gemäß einem der vorhergehenden Ansprüche,
wobei in Schritt e) die Bearbeitungsbefehle Informationen über einen Bereich von Interesse in der Visualisierung der datenreduzierten Bilddaten in dem Client-Gerät (10) umfassen, und
wobei in Schritt g) die hochaufgelösten 3D-Bilddaten in dem Bereich von Interesse als die bearbeiteten Bilddaten bereitgestellt werden.

5. Verfahren gemäß einem der vorhergehenden Ansprüche,
wobei die Bearbeitungsbefehle durch einen Bedienkontext, insbesondere ein Verharren in einer Ansicht, ein Überfahren eines Bereichs in der Visualisierung der datenreduzierten Bilddaten mit der Maus, ein Doppelklick und/oder ein Klick auf eine Schaltfläche, in dem Client-Gerät (10) bestimmt werden.

6. Verfahren gemäß einem der vorhergehenden Ansprüche,
wobei die Bearbeitung aus Schritt e) ein Zuschneiden und/oder eine Glättoperation der Visualisierung der datenreduzierten Bilddaten umfasst.

7. Verfahren gemäß einem der vorhergehenden Ansprüche, wobei die hochaufgelösten 3D-Bilddaten und die datenreduzierten Bilddaten demselben dreidimensionalen Koordinatensystem zugeordnet sind oder werden.

8. Verfahren gemäß einem der vorhergehenden Ansprüche, wobei parallel zu der Verarbeitung der hochaufgelösten 3D-Bilddaten in dem Serversystem (1) in Schritt g) eine Bearbeitung der datenreduzierten Bilddaten in dem Client-Gerät (10) durchgeführt wird.

9. Verfahren gemäß einem der vorhergehenden Ansprüche, wobei in Schritt c) zusätzlich Metadaten von dem Serversystem (1) an das Client-Gerät (10) übermittelt werden, wobei die Metadaten Informationen über die datenreduzierten Bilddaten und/oder darüber umfassen, welche weiteren datenreduzierten Bilddaten auf dem Serversystem (1) zur Verfügung stehen.

10. Verfahren gemäß einem der vorhergehenden Ansprüche, wobei der Schritt a) eine Komprimierung und/oder eine Zerlegung von ursprünglichen Bilddaten und ein sequentielles Hochladen von den komprimierten und/oder zerlegten ursprünglichen Bilddaten auf das Serversystem mittels eines Uploaders umfasst, um die hochaufgelösten medizinischen 3D-Bilddaten auf dem Serversystem (1) bereitzustellen.

11. Verfahren gemäß einem der vorhergehenden Ansprüche, wobei das Verfahren vor Schritt c) ferner die folgende Schritte umfasst:
Bestimmen von Patienteninformation auf Basis der hochaufgelösten 3D-Bilddaten, insbesondere mittels künstlicher Intelligenz,
Vergleichen von den bestimmten Pateienteninformationen mit den tatsächlichen Patienteninformationen, und
Ausgeben einer Warnung, wenn die bestimmten Patienteninformation nicht mit den tatsächlichen Patienteninformationen übereinstimmen.

12. Verfahren gemäß einem der vorhergehenden Ansprüche, wobei das Verfahren ferner aufweist:
Bereitstellen einer Visualisierung der hochaufgelösten 3D-Bilddaten in dem Serversystem, insbesondere basierend auf Metadaten der hochaufgelösten 3D-Bilddaten, und
Übermitteln der Visualisierung der hochaufgelösten 3D-Bilddaten von dem Serversystem (1) an das Client-Gerät (10).

13. Verwendung des Verfahrens gemäß einem der vorgehenden Ansprüchen in der Zahnmedizin, wobei die hochaufgelösten 3D-Bilddaten in dem Serversystem (1) Bilddaten eines menschlichen Kiefers umfassen.

14. Computerprogramm, das Befehle umfasst, die, wenn das Programm auf einem oder mehreren Prozessoren ausgeführt wird, den oder die Prozessoren veranlassen, das Verfahren gemäß einem der Ansprüche 1 bis 12 auszuführen.

15. System zur Analyse von medizinischen 3D-Bilddaten in einem Client-Gerät (10), umfassend:
ein Serversystem (1) mit einer ersten Recheneinheit (4),
ein Client-Gerät (10), das eine zweite Recheneinheit (11) aufweist und mit dem Serversystem (1) verbunden oder verbindbar ist,
wobei die erste Recheneinheit (4) und die zweite Recheneinheit (11) dazu ausgestaltet sind, das Verfahren gemäß einem der Ansprüche 1 bis 12 auszuführen.
